# EUROPEAN PATENT SPECIFICATION

(11) **EP 0 774 002 B1**
(45) Date of publication and mention of the grant of the patent: **02.07.2008**
(21) Application number: 95925667.8
(22) Date of filing: 26.07.1995
(51) Int. Cl.: C12N 15/34, C12N 7/01, C12N 15/86, A61K 48/00, A61K 39/235

(54) **DNA ENCODING OVINE ADENOVIRUS (OAV287) AND ITS USE AS A VIRAL VECTOR**
DNA KODIEREND FÜR DAS SCHAF-ADENOVIRUS (OAV287) UND IHRE VERWENDUNG ALS VIRALEN VEKTOR
ADN CODANT UN ADENOVIRUS OVIN (OAV287) ET SON UTILISATION COMME VECTEUR VIRAL

(30) Priority: 26.07.1994 AU PM710194
(43) Date of publication of application: 21.05.1997
(73) Proprietor: COMMONWEALTH SCIENTIFIC AND INDUSTRIAL RESEARCH ORGANISATION, Parkville, VIC 3052 (AU)
(72) Inventor: BOTH, Gerald Wayne, North Ryde, NSW 2113 (AU); BOYLE, David Bernard, Leopold, VIC 3224 (AU); VRATI, Sudhanshu, New Dehli 110 067 (IN)
(74) Representative: Lee, Nicholas John
(86) International application number: PCT/AU1995/000453
(87) International publication number: WO 1996/003508

(56) References cited:
- ADAIR B. M. ET AL.: "Serological identification of an Australian adenovirus isolate from sheep." AUSTRALIAN VETERINARY JOURNAL, vol. 63, no. 5, 1986, page 162 XP002083747
- ELGADI M. ET AL.: "Sequence and sequence analysis of E1 and pIX regions of the BAV3 genome." INTERVIROLOGY, vol. 36, 1993, pages 113-120, XP000561711
- SHIBATA R. ET AL.: "Nucleotide sequence of E1 region of canine adenovirus type 2." VIROLOGY, vol. 172, 1989, pages 460-467, XP002083749
- SALMON K. ET AL.: "Subcloning and restriction mapping of bovine adenovirus type 2." INTERVIROLOGY, vol. 36, no. 2, 1993, pages 72-78, XP002083750
- VRATI S. ET AL.: "Sequence of ovine adenovirus homologs for 100K hexon aassembly, 33K, pVIII, and fiber genes: early region E3 is not in the expected location." VIROLOGY, vol. 209, 1 June 1995, pages 400-408, XP002083751
- VETERINARY MICROBIOLOGY, Volume 41, No. 3, (1994), BOYLE D.B. et al., "Characterization of Australian Ovine Adenovirus Isolates", pages 281-291.
- NICHOLSON B.H., "Synthetic Vaccines", Published 1994, by BLACKWELL SCIENTIFIC PUBLICATIONS (OXFORD, UK), pages 346-361.
- JOURNAL OF APPLIED PHYSIOLOGY, Volume 76, No. 6, (1994), LEMARCHAND P. et al., "In Vivo Adenovirus-Mediated Gene Transfer to Lungs Via Pulmonary Artery", pages 2840-2845.
- BOYLE B. ET AL: 'Characterization of Australian ovine adenovirus isolates', Vet. Microbiol. 1994 41(3) p 281-91
- NICHOLSON B.H.: 'Synthetic vaccines', 1994 Blackwell Scientific Publ (Oxford) p. 346-361
- LEMARCHAND P. ET AL: 'In vivo adenovirus-mediated gene transfer to lungs via pulmonary artery', J. appl.Physiol. 1994 76(6) p.2840-2845

### SEQUENCE LISTING

(1) GENERAL INFORMATION:
   (i) APPLICANT: Commonwealth Scientific and Industrial Research Organisation
   (ii) TITLE OF INVENTION: DNA encoding ovine adinovirus (OAV287) and its use as a viral vector
   (iii) NUMBER OF SEQUENCES: 1
   (iv) CORRESPONDENCE ADDRESS:
      (A) ADRESSEE: Kilburn & Strode
      (B) STREET: 30 John Street
      (C) CITY: London
      (E) COUNTRY: United Kingdom
      (F) ZIP: WC1N 2DD
   (v) COMPUTER READABLE FORM:
      (A) MEDIUM TYPE: Floppy disk
      (B) COMPUTER: IBM PC compatible
      (C) OPERATING SYSTEM :PC-DOS/MS-DOS
      (D) SOFTWARE: PatentIn Release #1.24
   (vi) CURRENT APPLICATION DATA:
      (A) APPLICATION NUMBER
      (B) FILING DATE/ 26-JUL-1995
      (C) CLASSIFICATION:
   (viii) ATTORNEY/AGENT INFORMATION:
      (A) NAME:
      (C) REFERENCE/DOCKET NUMBER: P20032EP
   (ix) TELECOMMUNICATION INFORMATION:
      (A) TELEPHONE: 44171 242 8291
(2) INFORMATION FOR SEQ ID NO:1:
   (i) SEQUENCE CHARACTERISTICS:
      (A) LENGHT: 29544 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:

CTATTCATAT ATATAACGTT GCACAGAGGC GGGGCGTGTG GGTTTTTTAT TGTTTATTGT 60
CATGGAATTT ACAAAGAAGT AAGTTGTTGG ATCTTTATTC ACAATTCTTT TAACAATGAC 120
TTTTTTACTT ATTACATTTT TCATCTTTTT TACTTCACAT GATATTTTAC TTAAATTTTG 180
TACATACAAG CCAAAATTCG CATAAAATGT CTTACTTTAA AAAGTTAAAT TTTTTTTTTA 240
ACGCATAAAT GGACGTACAG CAGCAATTGG AATAGCAGGA AGGGCCATTG TAAAGTGTGT 300
TCCTGCTGAT GCCGCTGCAG AAAGGATAGA TGCTATCGTA CGCATAAACC CCCCTCCTAT 360
TTGTTCATCT GCTGCTTTTA TTATATCTTC TGCCAATCTA GGTGATATTT GCTTTTGAAT 420
GCTGTTTCCA AAAGCTTGCA TCATCGGATT TTCAATTAAA TGGATTGGAT TTGCAGAATT 480
TCCTTAAAAA TAGCCCAACC CATCTAAAGC AGTTAAAAGT ATTCTCCCTC CAGGAACCAC 540
AGATATAATT AAGCGGAGCA ACCGAGAGGT TAAATTCCAG GGTCCTCCGA AGAGAGTATC 600
TAGGATCAGG CCAAGAAGTG AACCAAAAAG ACTTGTAAGT AGAAGTTGTC TGATATGCTT 660
TGGAGAGGAC TGTTAAAATT GCAAAACGGT ATCTAATGAC CATTTCTTCT TTACTTTTAC 720
ATCTGTATCA TGTTCTCCAT CAGAAGGTCT TATTGGGAAG TACCATTGGT CACGAGCATC 780
TTTGAAGACT TCTGTTTCTT GAAATTCTGT TTTCGGTAAG CGACTAGCAG TTATGGTATT 840
AGGAATATTG ACGGTAATGT TATTCACATC TACAATTTCT GGAGGAATCC ATCTTGCATA 900
GGATGAAATG GGTTTTGTGG GTTCTTTCAA TATATAATTG CGAGGAGGGT TTTTCCAAAA 960
TCTCTGAACA TAAGTATTTT CTGATTTTGG CGGTTTTTTG CTTTTTCGCG CTCTTTTTCT 1020
TGGCTTTGGT CTTTGAAATT TTTTCTTCCT TTTTCTGTAG GCTCCTCCTG CTAAAGCTGT 1080
GTTATTTGTG ACGTACATCC TGTTAGCTAC ACGATTTTCC CGGACTGCAA ATTTTTTTGC 1140
CAAATGGAAA AGAAATTGCT GAAACCTTCT ATTAATCATA TAAATTGTCA GTGGAATCAT 1200
GAATCAGATA GTGCAGGATT TTTTCTTTTT GATACTGATA ATTTATACTA TTATGTATTG 1260
GATCAAGTGT CTTGGATATG TTTAAGAGAT ATAACTCTTC ATTGTGATCG CATGTGGTTA 1320
GCGGTTTGTT TTTGTTTGTG CAAATCTAAA TTTGATGTAC ACAATATTCT AGCGGGAGTA 1380
CATGTTATGT AATGAAAATG ACGTCGGGGA TTGAATGGAT TGAGCCTTAT TTGACATTTT 1440
TCTGTGATTT TTTTGCCTTA TTAGGAAATA AATTTGTGGC GCCAGTACGA TGGAGATTGG 1500
AATGACTCCT GCATTTACAG AAAGGAATTT GTACTGTGTT TTGCTTGACT TTAATTTAAG 1560
ATGGTATCAG CAGATATTTA ACCCAATATG GATTAAGCCA AATTTATGGG CTTTCTCTGA 1620
TTTTTTAAAA AAAATGGCCT TTATTTATGC TAGCGACTTG GCGTTGTTAA ATTCTTACAT 1680

CCCTGGTAAT GTTTGTAACA AACTTGATAT CATCAAGAAA GATCTTCCTG AAGATTTTAC 1740
CGTGTCTATG TTTTGTGTCT TAGTGTGTTG GCTTGCTTCT TTCTGTAAAG GTTCTAATTT 1800
AGCTGAAACT CGCCAGAATT GTCACGCGGT AAGCAAATTT CTGGCACAAC TATCAAAATT 1860
AATAAAACCC TAATTTTTAG TTTGTAAAAA TAGAATTCAA ATTTTTAACG CCACAATGAC 1920
TTCGGCGGAG TTTTCTGTTG AATTTCCTTA TGTTTCTAAG CCAATTGTTC CATGGCCTGC 1980
TTCGGCATCT TCTAATAATT CATCGAGTCA GAATATTGAC TTTCCTGTTC TTAAACCAGA 2040
TCAAGATCCA ATAGCCTTCT TTCAAACTAA CAATACGGCT TACTTACAAC CTGGAGCTAC 2100
TTATTACTGG AAGTGTATCG AACTGTCAAA GCCTATTCAC ATTTACGGTC AAGGAGCTAC 2160
AGTACAACTT GTCGGACCTG GACCTGTGTT TGTTTTCAAC AGTGAAAGTG TTATTCCTGA 2220
AGATTTTTAC GTCGTGTTTG AAAATATCAA CTTTATTGAA GATGAATTTC CTATTAGAAG 2280
TGGCCAGTTA AGTTTAGGAC TTACAACTCA CAGTGCTGTA TGGTTTATCA ATGTATGGAA 2340
AACTTCAATA GTCAATTGTA ACTTTAAAAA TTTTAGGGGA GCGGCTCTTT GGTATTCAGA 2400
TAATAGAAAT TTTTGGAATG CGAGAAAATG GAATCAGCAG CATTTAGTTT CAAATTGTCG 2460
TTTTAATGGT TGTAGAATTG GAATTTCTAA TACTGGTTCA TCTGAATATT CCATAGCCAG 2520
TCAAAATCAA TTTTATGATT GTCAAATCTG TTTTAATGTA ACCGGGGGTA ATTGGTCTAG 2580
AAATAATAAT GTTATTGTTA ACTGTAGATG TGCTTATCTG CATGTTGGAG ATAACATGTG 2640
GTATGAAGGC CATTCCGAAA ATAATAATCC CGCTAAGGGT ACTTTCTGCA ATAACATAAT 2700
TAACCATGCT GATAACGGAG GCAATGTCTG GCCTACTCAG TTTAAACTTA CAGATGGATC 2760
AACGATACAG TTAGCATCAT TTTATTTTGA TGATAATCAA GAAATTCCAC CTTGTTATAG 2820
CGGTAATTTT CATTGGTTTG GAGATGTAAA CATTGTAAAT TTTTCTACCA CAAAAATTGA 2880
TAAATGGTGC ATTACTGGAT GTAATTTCTA TGGTAATACA CATGCAGCTA ACGATGCTGG 2940
TCAAGTTCAG GTTGCTGAAG CTGTAAAAGA CAAAGTGTTT ATTATTGGGT GTTCTGGTAA 3000
TAATGTAACC ATGAAAAATA TTGTAGAAGG TAACATGACT CCAAAAATTG GTACAATAAA 3060
GTAAAAAACT TTTTATTCAA AACAAAATGG ATTTACATTT AAACGTTTTA CATATTGATT 3120
CTGCGTATAA GTTCTTTTTC TAAACACTCT TCTAATTTCC ATACATGCTT GATAAAACAA 3180
ACTTTGTAAA TTCATAAATA TAGGTTTGAC TTGATCAGAA GGTGAATAAT AGCTCCATCT 3240
AAATGATTCG GTAATAGGAA CATTATTATA TATTAACCAG CTATATTTTG AGTTAACTCT 3300

TGCATGATCC ACTATATCTT TAAGTACAGG GATAAGTGCA CTCGGAAATC CAAAAGAATA 3360
GTTTTTAATA AATCTATTTA TCTGTGAAGA ATCAAGCTGC GGACTAATAA CATGACATTT 3420
TGATTGAATT TTTAAATCCT TAATATTTCC TCTATCATGA CGCGGGTTCA TATTATGTAA 3480
AACTACTACA ACAGTGTAAC CATTACATTT GGCAAATCTA TTAAAAATTT TTGACGGTAA 3540
AGCATGAAAG AAAGAACTTA TAGAATGACA TGATCCCAAT TGATTCATAC ATTCATCTAT 3600
TATAATACAG ATAGATCCTT CACTTGCAGC TCTGCAGAAT ATATTATCTG GATTATCAAT 3660
ATTTAGATTA GTATCGGAAA TAGCATCTTT GAAAGCTAAT TGTATAAATT TTGGATTTAA 3720
TGTTTTTGTT AGTGGATTAG AGAATGCATC GTAGTTTCCT TCAACACACT GTGCTTTCCA 3780
CGCAATTTTT TCTTCTAATG GAACAGTACC TTTTTCTGGA GTTATGAAAA AAATTGTTTC 3840
TGGTATTGGA TCAATTAGTT TTCCAGATAT AATATTTCTT ATAAATTGAG ATTTTCCGCT 3900
ACCTGTGGGT CCATATACAG TAACAATGAA TGGTTGTAAT CCGCAGTTTA AACTGGGTAT 3960
ACAGCCATCT TTTAACAGAT TGTGAGCCTC ATTTACAGTT TTTGATAATT TTACAGCAAT 4020
ATTGTGTAAA TCAGTCATAA GTTGACCATG ATACATACAT TTATCAAAAA CTTCTTGACT 4080
TTCTGGAAAT GGATTTCTGC AAATAGAAGG ATCTATCTTT ACAACATCAT TTTTCCAATT 4140
TAATGTGTCA CTTAAAAATT TTCCCAAAAA GGATTTTCTG TCAATGGTTC TTGCGGTCTT 4200
GGATTTGGGT GTCTCTTGTC GTACGGGTAA AGTAAGTATC CTTTCTTCCA CTGGATCCCT 4260
TTCCTCATCG TTTGATCCTT CCAAGGTCTC AGAATTCTGG TTAGTTGCTT CTCTACCACC 4320
GTGAATGGTA CATCGGTTCC ACTTGCGGTT TGCAGTGTCT TTTTTAAACT TTTCCTCGAT 4380
GTCTGAAACT CTTTCTGTGG TTGTTCTAAT AAATTATAGT CAGTAAAACA ATGTTTTAGA 4440
ATTTCATAGT TTAAACAATT TTTAGCATGA CCTTTGGCTC TTAATTTTCC TTCTCCAATA 4500
AATTTACAGT TTTTACAAGT TATGTCTTTT AAAGCATATA ATTTAGGAGC TAAAATACAT 4560
GTTTCTGAAC TGAATGCTTC AGCTCCGCAA CGGTTACAAA CAGTTTCGCA TTCAACCAAC 4620
CAAGTTAGAC ATGGATGTTT TTCATCAAAG ATTAAATTTG AGTTATATTT TTTAAGTCTA 4680
TGTAATCCTT TTGATAACAT GAGTTGGTGG CCCTTTTCTG TTAAGAATAA CGAGTCTGTA 4740
TCACCATAAA TACTTTTTAT CTCCCTTTCT ATGTAAGGTT TACCCATATC TTCCCCATAT 4800
AAAATTTCTG CCCACTCACT CATGAAAGCT CTGGTCCAAG CCAGCACAAA GGATGCTATC 4860
TGAGTTGGAT ATCGGTTGTT CTTGATCCAT TCTTCCTTAT CCTCAATAGT TGTTAAAATT 4920
AAATCATTAC AATCAGCAGA TAAAAAAGTT ATAGGCTTAA AAGTCACGTG ATCTTGATTT 4980

CCTATAAAAA GTGGAAAATT AAAATTTTCA TTTGTGTCTT TGGAATCTTT GGGCGGCATT 5040
TCAGGTAGGT TTGAAAAATA CTGATTCCAC TCAAATGAAC GTTTTGGTAA TGATTTACTA 5100
ATCACAGTTG TGTATGATGT AATTTCAGCT GATCCATTTT CTAATCTTTT TTTATCTTTC 5160
TCTTCAATAT TTTCAGCAAA CACTACTTTC TTTTTATCTA TACGGGTAGC AAACGAACCA 5220
TATAAAGCAT TTGATAACAA TTTACTTATA CTTCGCTGAA TCTTGTTGTT ACTTTTACTT 5280
GCTTTTTCTT TAGCCATAAT ATTTACTTTC ACATATTTTT GACATAACGG TTTCCAGTCA 5340
CTCCATACAG CATACATTTC AGAGCTTTTG ATTATTTTGC ATTTCCATCC TCTATTGTGT 5400
AAGGTGATTA AATCGATAGA GGTCAGTACT TCATTTATCA ATGTTTCATT TGACCAGCAT 5460
AACTTTCCAC TTTTTTTAGA ACATAATGGA GGTAACACAT CAAGATAATC TAATGATGGG 5520
GGTTCACAAT CGGCTACCAC AATCATAGGT TTGATTGAAT TGTCAAAATA ATCTATTTTT 5580
TCTTTTCTTT GTAGTAGTTC TTGAAAGTAA TCTATTTGTG CATTGGCTTC AAAAGCATTT 5640
AAAGTTTTTC CATATGGAAG TGGATGCGTT AAGGCACTAG CATACATTCC GCAGATATCA 5700
TACACATATA TTGCTTCTTC AAATATTCCT AAAAATGAAG GATAACATCT TCCTCCTCTT 5760
AAACTCATTC TAACAAAATC ATACATTTTT TCTGATGGAG CTTCCAAATT TCTTAGGAAT 5820
TCAGAGGGAT GATCTTCTTC ATTATAAAAG ATTTGTTTAA ACAATGCTTG AGTATTACTA 5880
CTAATTGTAG GACGTTGGAA TATATTAAAA GAACACTCAA GCTTTAAAGA TGTTGTACAG 5940
AACTCTTGAT AACCTTCTAT AAGTTTTTCA ACTAATTGAG CCGTAACTAT AACATCATCA 6000
ATACAATACT CCTTAGCTTC CTCTAATAAG TTGTATTTTT GGTTGTGTTT TGGTTTGTTT 6060
TGTAAATATT CTTCAAATGA ATTCCAATAT TTTTGAACTG GATAACCATT GTTTTCTTTT 6120
TCATATTCTC CCAACATAAA AAAATCATTG ATTGCCCTGT AAGGACAATA ACCTTTGCTA 6180
ACACTCAACT GATATGCAGT AGCAGCGTCT CTTAAAGAAG AGTGGGTTAA CAAAAATGTA 6240
TCCCTAACCA TAAATTTTAT ACCTTGCCAT TTCATATCTT CAAAATTAAT AATTCCATTT 6300
TTCCATCTTT CATAAGTTGT ATGTGAAGGT TTCTTAAAGC AAGGATTTGG AAGAGATAAT 6360
GTAATATCAT TAAATAACAG TTTTCCAGCA CGAGGCATAA AGCTTCTTGT CAGCTTAAAC 6420
ATTGAAAGTT CTTCACTGTC TATTCCTTCT AATACATGAC TTGCAAGTAT GATTTCATCA 6480
AAACCACAGA TATTATGACC TACTACATAT AATTCAATAT ATCTTGGTTC GCACTGTTTT 6540
AATTTTTTTT CTTTATTTAA GACCATGATG TCTTCATATG ATAAATTTGA TTCAAGACCA 6600

TGATTTTCAC AAAACGTTGA CCAGTATTTT TTAGCTACTG AAATTTGTAG CTCTGTTCTG 6660

AATTTTTTAA AAGCTATGCC AATTTCATCT TCTTTTTTAT TTAACATTAC AAAACATTCT 6720

CTGTTTACCT CATAACCTAT ATCGGTAGCT ATTTTAGAAG CAATTTTTAT GAGTGATTTA 6780

CATCCAATTA ACTTAAAAAC CAACAAGTAA GGAGTTAACT GTTTTCCATA CAAAGAATGG 6840

TAAGTATATG TTTCAATATC ATAAACAATA AAAAGACGTT TTGCTTTTAT GGCTCCAACT 6900

GGATTAAATT TGATTTTTTC CCACCAGAGT TTTGTTTCAT GGTGAATATT GTGATAATAG 6960

AAGTCCCGTC TTCTGGATGA GCAGTTGTGT ATATTACTAT AAATTGTTCC GCAGAATTCA 7020

CATTTATTCT GTTGTTTAAC AGTTTTTATT AAATATATTT CTCCTTTTAA AATCAATAAT 7080

TCTATTGGTA ACAAATTTCC ATTAAGAATT TCTTCAGTCA TCTTAAAAAA TCTTTTGTTG 7140

AACTTCCATA TTTTTAAAGA TACGGGGGTG TTAGAATCAC AAAGTTTTAA AACATCTAAA 7200

ACATTTTCTA CTTTCTTGAA AGAATTTAAT TTTAAACCCT GAATTGCAAA GTAATTATAA 7260

AAACTTTTTT CAAAATTCTT GTAGTATATA ATTTTTATAT ATGTATCCTC ATATATTCCA 7320

GTAATATAAG TAGTAGTTCT TTGCTTTATT ATTGTCTTTG AAGCCATCTG TTTAAAGCCG 7380

CTTCCCGTAC TCGCTCAAAG CTTCTTAAAA CAACTTCATT TGTACTATAG CCAACAATTC 7440

CAGACAATTT TATTCTAAAT GCTATTTCAA CTGAATCTAA ATCTGAAAAA TCCGTGTTTA 7500

CTTGGTTGAT TACTTCTTCT ATGCTCCCAC TGTCTTCTAC GAAGTCTATA TCTTGAAGTA 7560

ATTGGTCTCT TTCTTCTGGA GTTGAAAAAG AGTAAGATCT TTCATTAGCT TCTATAATTC 7620

CTAAAAAATC ACGAGTTATT CTGCTATATA GTTGTCTGAA TGCTTGTGTT TCTCTATTAA 7680

ACCAAACTCT AGTAAATATA TCTTCTCCAT TTTCATTTCT ACCTCTTAAT ATAATTTGAA 7740

CAAATTGGAT TCCAATATTT CTGGCAGCTA ACCTATTTTG CACTAAATTT AAGTATAAGT 7800

AATATAGCGT GCTTGCCACA TGCTCTAATA TAAAGAAATA CACTAACCAT TTTTGAATAA 7860

AATCATCAGT CAATCTATTT TCATTATAAA ATCTAATAAG TAATTGAAAA AATTCACTTC 7920

CGTAATTAAA AAAATTACTC CTTCTTGCTT CAGGAGTTAA TTCTTCTTCT AAATTTTGAA 7980

TTAAATCTAC TATTGAAGCT ATCACTTCAT CATTAAATTC TTCCCTACTC AGATCGCTTG 8040

AGCTCGGCTC GCGATCTGAA AATCCTTCAT CTTCTATTTC AGGAACAGTA AGAGGAGAAC 8100

TAGAAGTTTC TTCAACATTC CTTACCCTTT GGCGTCTATT AACAGGTAAT CTATCAATAA 8160

ATCTTCTGAT TACATCACCC CTTGAACGTC TCATTATTTC AGTAATAGCT CTATAATTTT 8220

CCCTAGGTCT TAATCTGAAT GGTAATCCTA CTCTTGTCCC TGACCTTAAA GTTAATGCTC 8280

CACCATGCAT CCCACCTTTT CCTAAAGTTA ATACAGTTGC TAAATCTTTT AAATTAATTC 8340
GATTTTCAGC TTCTGGAATT TCCAGCTGTG AAAATTCATC TATAAAAAGC TCAATCCAGA 8400
ATTCAGAAAA AGGTAAGTCT AATATACATT CACTATTATG CATGTTAGAC AAAATTAAAA 8460
ATTTACATAA AGCTTTTTTA ATTTTACAAA TTAACTTTAT AAGGTAAGTA TCCCTTTCTT 8520
GCAAATTTAA AACCATAAAA GCTTGAGAAA AAGGTTGATA ATGCTGCTGA AAAGATCTAT 8580
TCTGATTTTG AGCTGAAATA GCGGAGCCAA AACCTTGCAT GTCTGCAAGT TGCAGACTCC 8640
CTAATATTCT ATCCATTAAA ACCGCGTTTT GAATTTGACT AATTGTTTGT GAAAAATTTT 8700
CTACATTTTG AATTGCTCTC ATATATGACC CAGTATTTAT GGAGTATGAA CAATCAGTTA 8760
AAATTTGCCA GGTCATGCGT CTCTCAAAAC TTATAGGTGA AAGATACAAC TTATATGAAA 8820
TGTTGCTGTA AGTCCGCTGA TCAAACAGAT ACTGGTTTAA AACTCGCGCC ACATAAAAAT 8880
ACCCAATTAA TAAATTTGGT GGAGGTTCTC CTTCAAATGG TGGTTGTGAA GTAACAGGTC 8940
CTCTTGGGCG TAAATCGAGT AATTGAGTCA CTGGATAATT AAAAAATCGA TTAGCCCATT 9000
TTATTCCCCT TTCATGTATA GTCCTTGACC TGGCAATACT TCGATTATTA AGGTCAAGTG 9060
TTAAACGTAA ATATCGTAAG GTATGTTGAC TTTGCCCAGT GAGTTGTTGC CATTGGTGAA 9120
TCTGCAAGGC AAACAAAAAA TTTATCTTAT TACTGCAGAT GCATCCTATT TTACAAAATT 9180
TACGTTCATC ATTGGAAACT CCAGACTTAT CAAGCAACTC CCCGGGCACG TCAAATAAAA 9240
ATGAAAAAGA TGAATTTGAA CCAGCAGTTG GCATTTCTAG CAAACCATCT GATGAATTTA 9300
ATATGAGACG ATCTCAAAGA GATGATAATT TACCTAAAAG TCAGATACCA GTAGTAGATA 9360
TACTACATGA TAAAAATCCT AAAATGGCAG AAGAACGAGA CTTAATGTAT AAATCTTCTG 9420
CTTGCATAAA ACTTGATGAT TCTAAACAAT TAAAAACTGA TATGTTCAGG CCGGATTTTG 9480
CTGGAACTAG TCCAGCTCAA AGACACATAG AAGCCGCAGA GCTAAAGAGA AATGGATCTT 9540
ATACTCGTAG TTTAGAACAA TGGACACATG ATTCTTTTAT AAGTCATGTT AAACAATTAC 9600
TTTCTAGACC ATTTATATCT CTAGGTATTA CATATTTGGA TGATTTTTTG CAGACTTATT 9660
TAGATCATAC TGAATCGTCT TCTTTAAACT TTCAACTGTT TACTTTAATA AATCACTGTT 9720
CAGAAAATAC TTTAAAACGG ATTTTAAAAC ACATTTCTAA AAAAAATGAA AAAAATCAAT 9780
ATGTAAATCA ATGGTTGATT GATCTCATTA CATGTATATA TCTAATTATA AGAGATGAAC 9840
AAAATGTTAC AGAACAAGTT AATGCCCTTT TAGTAACTAG TAATCACTTA GCTTTACATT 9900

TTGCAAAGAA AGCTACAGGT GGATTCTATC CTACAGCAGA CAAGTTAGCG AAGACTCATA 9960
TTTTTTTCAA GAGAATAATT TTAGGAATAC TTTCGCTAGC AGAAAGTATA GGTTGCTATA 10020
CTGTGAATCC ATATTGCAAA AATCCTTTGA AAAAGTCAAA AGTAGAAGTA GAACCAAGTG 10080
ACGAAATGTA TATGTTCAGC TTAAAAGGTG CACTTGAACA TCCTGATTCC GACGAAGACG 10140
AAGACAGTGG ACTTCAAAAT GAATAATTAT CATAAATGGA CTTCTAATGT TATAGATGCA 10200
ATTCTATCAA ACAAAGCTCT TTTAGCTATA AAAATTTTAA AAGTCAACCG TTTGCAAACA 10260
AATTGAATGC TTTAGAATCA GCAGTTGTGC CTCCAAGAAA AGATGATACT CCTGAAATGA 10320
TAGCAAATCT TTTAAAAGAA TTAGTTGCTT TGGGAGCTAT TCGCAGTGAT GAAGTTGGCC 10380
CATTATATTC TGACCTTCTT ATCAGAGTTC ACAAATATAA TAGCTTGAAT GTTCAATCAA 10440
ATTTGCAAAC TTTAACAGGA GACATTAAAT CACTTCAATC CGATATAATT AGAAGTTCCG 10500
ATATTCCCAA TTTAAGTAAT CAAGTTGTTT TAAATACATT TTTAAATTCT TTGCCCTCAA 10560
CTGTTACATT TGGACAACAT AATTATGAAG CTTTTAAACA AACTCTAAGA TTATTTGTTA 10620
ATGAGACACC TAATATTACA GTTTTTAGAT CAGGAAATGA TACTTTAATT CAGGTTAACA 10680
TAACAGGAAT TCATACAATT AATTTGAATG ATGCATTTAA AAATTTAAAA AATTTTTGGG 10740
GAATAGTATT AACAGGTGAA TTTATTCCAG GTGATATTAC AAGCAGACTA ACAGCTAATA 10800
CAAGAGTACT GCTTTATTTT CTTGCTCCTT TTACAAATGA TAATACATTC ACACCTGATA 10860
CTTTTCTAGC TTTACTCATG AAATTATATA GATTGACAGT TTCTTCTGCT TTAGATTTTG 10920
AAGAAGAAAC TGAAGCTGAA GTAGAAAATG TAGCTCAACA AATAGGATCC ACTAGTGCAG 10980
ATTTTACAAA GACTTTAGGA TATCTATTAA AAAACAAAGA AGAATCATTT TCGCCTCCCA 11040
AATCATTATC TCCTAGACAA CTGGGTATTT TAAGGTTCAT ACAGAAAAGT CTGGTAGATA 11100
AAATTGATAG AAATAATGAA GATCCATGGG ATGCTTTAGA AACTTTATCT TATTCATTTT 11160
CTCCGTCATT TTATGAGGCC AATGGGCCTT TTATTAGACG GTTAATAACT TATATGGAAT 11220
TTGCCTTACG TAATTCTCCT ACTTACTTCA GAGAAATTTA CTCCAACAAA TATTGGATAC 11280
CACCCAATTC ATTTTGGACT CAAAATTATG CAGACTTTTT TTCGGAAAAG AAAGAAAAAC 11340
AAAATTTCGA AACATTTGAA CCGCGGGAAC TTCCTTTACA AATCTCTGAG GAAGAAGCTG 11400
TCCCGCATAC AGAAGATTTT CAGTCAGCCA TCTCGCCCTC TATGGGCCAA ACTTCACTCC 11460
CTGCTCCTTC TGTGTCAGAA TACAGTAGCG TGCCTCGGTC AGCTTTTTAC CCTCTCAGAG 11520
AACGTATCCA AGAGAGCATT TCAAAGGCAG TCATCCCTCC TTTGACAGGC TATGTCGGAA 11580

AACAAATAGG TGAAACTATT TTCCCTGGTA GTGGAGATCT TGTAGCACCC GCTGCGTCTT 11640
TAGTTGCAGC ACAATTGGTT GATTCAAGGT TTAATAACAG AAGACAAAGA TTGAAAGACG 11700
CAGCCAGAAA GCGTCACCGC TATGTTAGAG AGATGCATAA TATTTCTGAT AAAGAGTCAA 11760
ATGCTTCTAA TGATACGGTA ATATCACCTT TGATTGGACA TGGTTCGCGC ACTGAAAATC 11820
GTTTTGAATA TTTGAGACCT AAAGGTGGAA ATTATTTATA CTAATAAAAA TCATAACAGA 11880
CCTGACGGGC GGTCATCCTT TTTTATTAGA TGCAGAAATT TGTACCTCCA CCACGAATCC 11940
TTGCTCCAAC AGAGGGTAGA AACAGTATTA CTTATACGCC TCTGGCACCA CTGCAAGATA 12000
CAACAAAAGT ATTCTTTATT GACAATAAGT CTTCGGACAT TGAAAGTTTA AACTTTACTA 12060
ATAATCACAG TAACTTTTTT ACAAATATTA TTCAAAATGC TGATTTGGCA GCGGATGAAG 12120
CAGCAACGCA AGATATTAAA CTGGATGAAA GATCTAGATG GGGCGGTGAA CTGAAAACTT 12180
TTATAAAAAC AAATTGCCCC AATGTTTCAG AATTTTTTAA CAGTAATAGC TTTCTAGCCA 12240
GATTAATGGT AGATAAAACT GATCCAGAAC ATCCTAAATA CGAATGGGTA CAAATTACAA 12300
TTCCTGAAGG CAATTACACT GGAAGCGAAC TTATAGATCA ACTTAACAAT GGTATTTTAA 12360
ACAATTACTT AGAAGTGGGA CGCCAAAAAG GAGTAGAAAT TGAAGACATA GGAGTAAAAT 12420
TTGATACAAG AGATTTTTCA CTTGGATATG ATCCTGAAAC GGGACTAATT ACTCCAGGAA 12480
AATATACATA TAAAGCTTTT CATCCAGATA TTATCTTGCT ACCTGAATGT GGCGTAGATT 12540
TTACATATTC TAGAATTAAT AATATGTTAG GTATAAGAAA GAGATTTCCA TATACTAAAG 12600
GATTTCAAAT TTTATACAGT GATTTGACGA AGGGAAATAT CTCTCCATTA CTGAATTTAA 12660
ATAACTATCC TCATTCTATC GAACCTGTAA TGCAAGACGA AAATGGAGTT AGCTATAATG 12720
TAGAAAAAAT AAGTGACAAT CCCCCCAGAT GGCAAACAAA GTACAGATCT TGGACTTTAA 12780
GTTATAAAAA TAATGGAGGA GCTAAAGCCC TAACTGTACT AACTGTTCCG GACATAACAG 12840
GAGGATTAGG TCAAATTTAT TGGTCAATGC CAGATACTTT TAAAGCACCT ATTACTTTTA 12900
CTAACAATAC TACAAAGCCA GAAACACTTC CAATTGTTGG ATTACATATG TTTCCTTTAA 12960
AAGCAGGGTT AGTTCATAAT ATAAATGCGG TTTATTCTCA ACTTTTGGAA CAAATTACAA 13020
ATACAACTCA AGTATTCAAT AGATTTCCTA AAAATGCTAT ACTAATGCAA CCACCTTACA 13080
GCACCGTAAC ATGGATAAGT GAAAATGTCC CCTTTGTTGC AGATCACGGG ATTCAGCCAT 13140
TAAAAAACAG CCTTACAGGT GTACAAAGAG TTACTATAAC AGACGACAGA AGGAGATCTT 13200

GTCCATACAT ACAGAAATCT TTGGCGACTG TTGTCCCTAA AGTACTTTCA AGTGCTACAC 13260
TTCAGTAACA ATCTGGCTGA TATCTCTGGG CCTTATCCTC CTGGAACCGT TATGTCTATT 13320
TTAGTTAGTC CCTCTGATAA TACCGGGTGG GGTATTGGAA CATCAAGTAT GAGGGCTACT 13380
GGCTTGAAAT TTTCTAAAAA ACAACCTGTT AGAGTGCGAC CTTATTACAG AGCTCAGTGG 13440
GGACAGCTTA ATGCTCGTAC TTCACTTGAG AAACTAAAAA CCAAATTGAA ATATTATGAA 13500
AAATTGTACA GGGACAGACT AAAAAGAAAA ACAGTTGTTC CAAAGAAAAA GAGGTCACCT 13560
ACATCTCCTG CGGATCGACT TAAAAAATAT CTTAAAGCTG TCAGTCAAAT CAAAGCTTTC 13620
AATAGAGCTA GAAGAGCAGC CCAATAAATA TTATTTTTCA CTTGCAGATG AAGGTAGTTC 13680
ACGTGCTTAA ATCTCCTCAT CGTCGAAGAC ATACACGTCG TTACAAAAAA CTAAAAAAAA 13740
TCAATCTATC TCCATACATT TTACCTAAAG AATTGCAAGG CGGTTTTTTA CCAGCTCTCA 13800
TTCCTATCAT AGCAGCCGCA ATTAGCGCAG CCCCTGCTAT AGCTGGAACT GTAATAGCTG 13860
CTAAAAATGC TAATCGTTCT TAAAATTTAG AAAACTTTTT TTTTAACAGA TCACATGGCT 13920
TTTTCAAGAT TAGCTCCCCA TTGCGGCTTA ACACCTGTTT ATGGCCACAC CGTTGGAATC 13980
TGTGATATGA GAGGAGGTTT CAGCTGGTCT AGTTTGGGAA ATTCTTTTAC TTCTGGTTTA 14040
AGAAACATAG GTTCATTTAT ATCAAATACT GCTCAAAAAA TAGGTCAATC ACAAGGATTT 14100
CAGCAAGCCA AACAAGGTCT ACTGCAATCA AATGTTTTAG AAAATGCAGG ACAATTAGCA 14160
GGTCAAACTT TAAATACTTT GGTAGATATT GGAAGATTAA AGGTAGAGAA AGATCTAGAA 14220
AAATTGAAAC AAAAAGTTAT AGGGAACGAC CAACAAATTA CTCAAGAACA ATTAGCTCAA 14280
CTAATAGCCA GCTTAAAACC AAAAGATGAA ATGTTTGTAA AGCAATCAGA AAAAATTGTT 14340
GAACCTATGA GACCAGAAAT TAAATCTAGC CAAATGCCTG TAGAAATGTC TTTTTATGAT 14400
TCTGTAAGTG ATGAACCAAT CATAAAAACC AAAGAAGTTA GCCCTCCTTC ATTTTCATCT 14460
GAATCTTCAC ATTCATATTC TCACCCAAGA AAAAGAAAAC GCGTATCCGG TTGGGGTGCA 14520
TTTTTGGATA ACATGACTGG AGATGGAGTA AATTTTAATA CAAGAAGATA TTGTTATTAA 14580
AAACACTTTT TATTTACAGA TGGAGCCACA GCGTGAATTT TTTCACATTG CGGGTAGAAA 14640
TGCAAGGGAA TACTTGTCTG AAAATCTGGT ACAATTCATC TCTGCCACTC AAAGTTTTTT 14700
TAATCTTGGA GAAAAATTTA GAGATCCTTT TGTAGCTCCA TCGACGGGTG TAACTACTGA 14760
CCGTTCTCAG AAACTTCAAC TTCGTATAGT TCCGATTCAA ACTGAGGACA ATGAAAACTT 14820
TTACAAAACT AGATTTACTT TAAATGTAGG AGATAACAGA GTTGCAGATC TTGGAAGTGC 14880

ATATTTTGAC ATTGAAGGAG TTATTGATAG AGGACCTACT TTTAAACCTT ATGGAGGGAC 14940
AGCTTATAAT CCATTAGCCC CAAAATCAGC TTTTCCCAAT GCAGCTTTTA TGGATACTGA 15000
TGAAGCTACA ACAATTTATA TTGCTCAACT CCCTAATGCT TATAATGCTC AAAACAAAGG 15060
TGTAGAAGAA GCAATTCGAG TAGAAGCAAA CACTACTACT CCTAATCCTC AATCAGGAGA 15120
ATATGCTACT TATGACTCTG CCAAATTTAA TCCAGAAACT ACTGGTGCTT CTGGAAGGCT 15180
TTTAGGAATT AATAGCTTAG GAGATCTTTT TCCGGCTTAT GGATCTTATT GTAGACCTCA 15240
ATCAGCAGAT GGTAACATTT CAACTGCACC CATAACTAAA GTCTATCTAA ACACTACTGC 15300
TACAGATGAC AGGGTCAGTG GAGTTACTGC AGTTGACACC GCAACCAGAT TGCATCCAGA 15360
TGCTCATTAT ATTGAATATA CTGATGAAGC CAAAGCTACA GCTATAGGAA ATCGCCCAAA 15420
TTATATTGGT TTCCGAGACA ATTTTATTGG ACTCATGTTC TACAATAATG GTTCTAATGC 15480
AGGAACATTT TCCAGCCAAA CACAACAACT TAATGTTGTT TTAGACTTGA ATGACAGAAA 15540
CAGTGAACTA AGCTATCAAT ATCTAATAGC AGATCTGACA GATAGGTATA GATATTTTGC 15600
ACTTTGGAAC CAAGCAGTTG ATAGTTACGA CCAGTATGTC AGAATTTTGC ATAATGAAGG 15660
ATATGAAGAA GCCCCTCCGG CCTTATCATT TCCTTCTCAA GGTATCCAAA ATTATTTCAT 15720
GCCTACTGCG GCAGGTAATG CGATGACAGT AGACACGGGT AGAAATACTG CAGCAAAAAC 15780
AGATAACACC AAGGCTTTTA TAGGATATGG CAACATGCCA TCTTTGGAAA TGAATCTGAC 15840
AGCAAATCTA CAACGTACAT TTTTGTGGTC TAATGTAGCA ATGTATCTGC CAGATAGGCT 15900
GAAAACAACA CCACCCAACA TAAATCTACC TGATGACACC AACTCTTACG GATATATAAA 15960
TGGAAGGGTC CCTCTAGCAA ACATAATAGA TACATGGACT AACATTGGGG CTAGGTGGTC 16020
ATTAGATGTT ATGGATACTG TAAATCCATT TAATCACCAC AGAAATTCAG GACTAAAGTA 16080
TAGGTCACAA CTGTTAGGAA ATGGAAGATA TTGCAGATTT CACATTCAAG TACCTCAAAA 16140
ATTTTTTCCT ATAAAAAATC TTTTGTTGCT GCCAGGAACA TATAATTATG AATGGTACTT 16200
TAGAAAGGAT CCCAACATGG TTTTTCAGTC TACTTTAGGT AACGACCTTA GAGCAGATGG 16260
CGCAACTATT ACATACACCA ACATAAATTT ATATGTTTCA TTTTTCCCTA TGAATTATGA 16320
AACAGTAAGT GAACTTGAAT TGATGTTGCG TAATGCTACT AATGATCAAA ACTTTGCAGA 16380
TTATTTGGGT GCGGTAACTA ATCTTTATCA AATCCCAGCT AATACAAATA CTGTAGTAGT 16440
GAACGTACCA GATAGATCTT GGGGTGCTTT CAGAGGATGG AGTTTCAATA GAATTAAAGC 16500

TTCAGAAACA CCTATGATAG GAGCAACAAA AGATCCAAAT TTTACTTATT CAGGATCTAT 16560
ACCGCTACTA GATGGTACTT TCTATTTAAC ACACACTTTT CAACGAGTTT CTATTCAGTG 16620
GGATTCTAGC GTTCCATGGC CAGGAGATGA TAGGCTTTTG ATTCCAAATT GGTTTGAAAT 16680
TAAGAGAGAT CCTAATATGG ACGCAGAAGG TTATACTATG AGTCAAAGTA CTATCACAAA 16740
AGATTTTTAT TTGGTACAAA TGGCTGCTAA TTATAATCAA GCTTATCAAG GTTATAAATT 16800
GCCAGTACAT TCTAAATATT ATGGATTTTT AGAAAATTTT CAACCTATGA GTCGCCAAGT 16860
ACCAATTTAT GGTAATGGCA CTTATGATTT ATATACTGCT TATATTACAA ACCAAAGAAC 16920
CATGCAAATT TGGAATAATA GTGGTTTAGA ATCTAAAACT TCAAATCCTC CTATGTTATC 16980
CAACACTGGT CATCTTTATG TAGCTAACTG GCCATACCCT TTGATTGGAC CAAATGCTAT 17040
TGAAAACCAA CAAACTGAAA GGAAATTTTT GTGTGATAAG TATATGTGGC AGATACCATT 17100
TTCTAGTAAT TTTTTGAATA TGGGTAATTT AACAGATTTA GGGCAAAGTG TTTTGTACAC 17160
TAATTCTAGT CATTCACTTA ATATGGTTTT TACTGTGGAT AGTATGCCTG AAACAACTTA 17220
TCTAATGCTT TTATTTGGTG TTTTCGACCA AGTTGTTATT AATCAACCAA CAAGAAGTGG 17280
AATAAGTGTA GCTTATTTGC GCCTTCCTTT TTCAGCTGGT AGTGCAGCAA CATGAGCGGC 17340
ACATCCGAAA GTGAGCTGAA AAATCTGATT TCATCATTAC ATTTAAATAA TGGATTTTTG 17400
GGCATTTTTG ATTGCAGATT TCCAGGTTTT CTGCAAAAAT CTAAAATTCA AACTGCTATT 17460
ATTAATACAG GTCCCAGAGA ACAAGGCGGA ATACACTGGA TAACATTAGC ATTAGAACCC 17520
ATTTCTTATA AGCTATTTAT ATTTGATCCA CTCGGATGGA AAGACACTCA ATTAATTAAA 17580
TTTTATAATT TTTCACTAAA TTCTCTTATT AAAAGGTCGG CCTTAAATAA CTCAGACAGA 17640
TGTATTACAG TAGAAAGAAA TACTCAAAGT GTTCAATGTA CCTGTGCGGG ATCGTGCGGC 17700
TTGTTTTGTA TATTTTTCTT ATACTGTTTT CACTTTTATA AACAAAATGT ATTTAAAAGT 17760
TGGCTTTTTC AAAAATTAAA CGGTTCAACC CCTTCTCTGA TCCCATGTGA ACCACATCTA 17820
TTACATGAAA ACCAGACATT TCTTTATGAT TTTTTAAATG CAAAAAGTGT TTATTTTCGA 17880
AAAAATTATA GAACATTTAT TGAAAATACT AAGACTGGAT TAATAAAAAC ACATTAATTG 17940
TATTCTTGCT TTTTGACGTT TTCATTAGTC TTCATCTTCA TCTTCTTCTT CACTGCTAGA 18000
TTCCAAGATG GTTTTTTTTT TCTTTGATGG AGTAGGCTCT TCAATAGTTC CAAAAGGATT 18060
CATATCAGAA TCCTCTTCTA TGTTAGGCAA CATAGTATTT TTAACCTGGA ATGACTGATT 18120
CCACTTAAAT TGAGAAAACT GAATTGGAAT GTTATTTCCC ATACATTCAT TCCAAAATTT 18180

ACGCACAAGA GTTAAACACT GTAACATATC TGGCAAGCTA ATTTTCATCT CACAAAATTT 18240
TCCATTATTA CGTCTCAAGT TGTATTGATA GTTACAACAT TGAAACACAA AAACAGCAGG 18300
GAATGTAACT GCTGCGGCCT GAACTCTATT AACATCCTGA ACATCAATTC CTTCCACTCC 18360
AGATATAGAA AATGGAGTTA TTTTAGGGAG TTGTTTTCCT ATTGTTTGTT TGCCACCATA 18420
ATTACATTCA CACTGACCCA ATATAAAAAG CATATTTCCG ACTTTAGCTT TCGGAAACAC 18480
AGCTTTTGTA GTTTCAATGG CATTTTGCAT AGCCAGCAAG GCCTTCTTTT CATCTGAAAA 18540
GTTAAGACCA CAACTGCGAG GAGAACATTG CCCAAAACGC TGATGGGCAT CCTCAGCACA 18600
TAACACGTAA TGTTCCTGAA CTATTTTTAC TACTTGTTTA TTCATACGCC CATTACTAAG 18660
AACACCCCTC CCTTCCTTTA GGGCTTGCAC CCCTGCTTCC GATGTTGGAG GCATTTCAAT 18720
TTCATTCACC CTTTTAAACA TGAAGTCACC ATGAAAACAT CTAGGACGGT CCTCCTCCCA 18780
ATCATGATAC CACAAATAAC AACCAGAAGC ATTAAAGTTT GGAATCAAGT CAATTTGCTT 18840
ACAAATTGCA CTATATAGCA TTCTACCTCC TACAGTAGCC ATAGATTTAC TGCTACTATA 18900
AGTCAAATTT ATAATTTTCA TCTTTTTCAT GTACTGAGCA AATAATTTTT CACAATCTCC 18960
TTCTTCAGGA TGAAACTTCA TTTGACTGGT ATCAACTTTA ACACACTCTC CAAATTTAGC 19020
TAAAATTTCG AGCGCCGCTT GAACTTTATT CTGAAATTCT TCTGTAGTAG ATTTTCTCTT 19080
CTTGATAGAT TTAGTAACTT TTTTAGAAGA CATTATGTTA GTTTTTTTCT CGTTGTAGGA 19140
TGGCTGAAAA AAATATGGGA GAGTCAGAGA AGGGTTTGAA CGAAGAAGAA TTTAACTCTA 19200
TTCTATCAAA ACATCTGGAA AGACAAATTA AAATCTGTAA AGCGTTAACA TCAAAATTAT 19260
CGAACTGGAA TATTGGAACA TTGTTAGAAA ACTTGTTATT TTGTCCTGAT GAAAGACAAT 19320
CATCAGGTGA TCCCGACCCA AAACTAAACT TTTATCCGCC TTTTTTAATT CCGGAATGTC 19380
TTGCATTGCA CTATCCATTT TTTCTAACAA CTCCTATTCC GCTATCATGC AAAGCGAACA 19440
AAATAGGAAC TAACACTTAC CGAAAATGGA TGAACAATCA AGTCCTGGAT TTACAAATAC 19500
CTTCCTTGGA AAATTGCAAA TGGGATGATA GCTTGGGAAA TGTAGATTTA ATTGAAGAGC 19560
TTAAAGAGAA CCAAAAACTT GTTTTAGTAA AACAAGACCA TGAAAGAAAT ATATGGTTTA 19620
AATCAAAATG CAAACAACTT CAAAGTTTCA GCTATCCCTC ACTCAGTCTG CCCCCAGTTT 19680
TACAACAAGT TTTAATTGAA TCTCTTATCG GCATTAGTCA GGATCCTAAT AACTTTGACA 19740
AAAATTACGA ACCTGCAATA ACTCTAGAAA AACTACAACA TGTAAACTGT GATCAAGATT 19800

TAAAACAAGT TCAACAAAAA GTATCTTCAG CCGCTACATA CGGAATACTT TTGAAATGCA 19860
TTCAGACTTT ATTCAGTGAC AAATTATTCA TTCAAAACTG CCAGGAATCA TTACATTACA 19920
CCTTTAACCA TGGTTATGTA AAATTACTTC AATTTTTGAC AAATGTCAGT TTAAGCGAAT 19980
TTGTAACTTT CCATGGTTTA ACACACAGGA ACAGACTCAA TAATCCGCAG CAACATACAC 20040
AATTGGCAAC CGAAGACAAA ATAGACTATA TCATAGATAC AGTGTATTTA TTTTTGGTAT 20100
TTACGTGGCA GACAGCAATG GATATTTGGA ATCAAACATT AGATGATAAA ACAATAAATA 20160
TAATTAAAGA GGAATTAAAC CAAAATTTTG AGAAAATTGT CAAAGCTGAA TCAGTTGATG 20220
AAGTTTCTGA AATTTTAAAG TCTATTATTT TCCCTGAACT CATGCTGCGA GCTTTTTGTT 20280
CTAATTTACC TGATTTTATA AATCAGAGTC AGATATCAAA TTTTAGAAAC TTTATCTGCA 20340
TTAAATCCGG CATACCGCAG TCAATTTGCC CCCTATTACC TTCAGATCTA ATTCCTTTAA 20400
CTTTCCTAGA AAGTCATCCA ATACTCTGGA GTCATGTAAT GTTACTAAAT CTTGCTTCAT 20460
TTCTAGTAAA CCAAGGCAAT TATTTGCATG AACCCGAAAA ACCTTTAAAT ATTTCATCAG 20520
TTTACTGTAA TTGTAATTTA TGCTCTCCGC AAAGAATGCC ATGTTACAAT AGCAGTTTGA 20580
TGCAAGAAAT ACTAACCATT GATAAATTCG AGTTCACAAA CTCTGATAAA ACAAAACAGC 20640
TAAAACTGAC CCTCCAAACT TTTGCTAATG CCTATCTTAA CAAATTTAAC TCAGCAGAAT 20700
TCTACCATGA CCAAGTTTTA TTCTACAAAA ACTGTAAAAG TAAATTTTCT AACCAATTAA 20760
CAGCTTGTGT AATAAAAGAC GAAAAATTAT TGGCTAAAAT AGCAGAAATT CAAATAACGC 20820
GGGAAAAAGA ACTCTTAAAA AGAGGAAAAG GAATTTATTT GGATCCAGAA ACAGGAGAAA 20880
TCTTAAACAA TGGAGAAGCC ATATCATCCT CTGAAAACTT CCAAAGGCAA AGAACTAGCT 20940
ATGCTCTACC ATCAAATGAA GGAGAGCGAG CTGGATGGGA AGCCGATGAG CGAAGAAGAC 21000
GAAGGAGAAG TGAGTGAGGA TGAAACAGAG ACAACAATTC CAAAGAAAAT GAAGTTTACA 21060
AGTAAGTAAG CTCTAAATTT TTTATATTAA AAACTGAATT TTTTTAGACA AAATTATTTT 21120
AAATTAAATC TTTATAGCTA GCAGTTGATC TTTGTTCGTT TTTCAGAAAA CTCAAGTGTT 21180
CAGTCATATC AAGTTCACTT GCCTCTGAAA CACGAAATTG CGGAAATTCT AGAAAAAATT 21240
AGACTAGAAT CTAAAAAATA TCCAGGAAAA GTTTATCAAA TAAGAAATAG AACTCCAGCA 21300
AGTATTACAA AACGATACCT GTATGAAAGA GATCTGAAGA AACTGTTCCA GTATCTAGAA 21360
GACGCAAAGA AGCTTTACGC TAAGTACCAA AGCTGAGGCT TTATAGTTTT AAATTTTCCC 21420
GCCATGGCTC AACCAGTGAC GCCTTACGTC TGGAAATACC AACCAGAAAC AGGATATACT 21480

GCTGGAGCCC ATCAAAATTA TAACACTGTT ATCAACTGGT TGCATGCCAA TCCACAAATG 21540
TTTGCCAGAA TTCAACATAT AAACACCGCA CGCAATGTTA TGGACAAATT CCGCTCTGAT 21600
TTGACCCGAG ATGACATCGC GGTTAACATC AACAACTGGC CTGCAGAGGA TTTAATGCAA 21660
CCTCCTAATT TTCCTTACAT TCCTGCGACC TCTAAATCCG CTTCAACCAT AAATGACTGG 21720
TTGGCTACCA CTCAAGGAAT TCAACTCAGT GGAACTAGTG AACTAAACGG GTGGGGATCT 21780
AACCGCCTGA CTTCCTATCC GGATATTCCA CCCATTTTAA AGTATGAAAG GCCTGGTCAA 21840
CAACTTCAAG GCCAAGGACT TTTTAAGCAA GAAAATATTC ATTTATTTTA CGAATCTCCG 21900
CGCCTCCCTC GCTCTGGAGG ATTAACTCCC CAACAATTTG TAAAAGAATT TCCGCCTGTT 21960
GTTTATAATA ACCCCTTCTC AGAATCTATG AGTGTATTTC CGAAAGAATT TAGTCCTTTG 22020
TTTAACCCTT CAGAATCTTT GAAAAAAACA TCCAGTCAAA CTTTACAATA TAAATAAAAA 22080
ACTTCTATTG ATCTTTATAC TTACACTAAA GCATCGCGTT TATTTTCGTC GCCATAAAAA 22140
TATATCAAAG ACCCGTAATT CTCTAACTTT AAATCATTTT TTGAACTAAT CTTAATCCAT 22200
TTAAATGTAG GAATTAATAT ATCAGAAACC AGTAACAAGC CAGAATTAAA ATATACTTGT 22260
GTCATTTTTA CAGATGAAGC GAGCACGCTG GGACCCGGTT TATCCCTTTT CTGAAGAGAG 22320
ACTGGTTCCT CTGCCTCCTT TTATTGAAGC CGGAAAAGGG CTAAAAAGCG AAGGGTTGAT 22380
CTTATCTTTA AACTTTACTG ATCCTATCAC TATAAATCAA ACCGGTTTCT TAACTGTAAA 22440
ATTGGGAGAT GGAATATTCA TAAACGGAGA GGGTGGCCTA TCAAGCACTG CTCCAAAAGT 22500
CAAAGTTCCC CTGACTGTCT CAGATGAAAC ATTGCAACTG CTATTAAGTA ATTCTCTAAC 22560
AACTGAGTCA GACTCTTTAG CTTTAAAACA ACCGCAACTT CCCCTAAAAA TAAATGATGA 22620
GGGGAGTTTA GTATTGAACT TAAATACTCC TTTAAATCTA CAAAATGAGA GATTGAGTTT 22680
AAATGTTTCA AATCCACTAA AGATAGCGGC AGATTCTTTA ACTATAAACT TAAAGGAACC 22740
CCTAGGATTG CAAAATGAAA GTTTGGGCTT AAATCTAAGT GATCCTATGA ATATAACTCC 22800
AGAAGGAAAT TTAGGTATTA AATTGAAAAA TCCTATGAAA GTTGAAGAAA GTTCTTTAGC 22860
CTTAAACTAT AAGAATCCTC TCGCCATTAG TAATGATGCG TTAAGTATAA ACATTGCGAA 22920
TCCATTAACT GTTAATACAA GCGGATCTCT AGGAATATCT TATTCTACTC CCTTACGAAT 22980
TTCAAATAAT GCTTTATCAT TATTTATAGG AAAACCTTTA GGATTAGGAA CTGACGGCTC 23040
TTTAACTGTA AATTTAACTA GGCCTCTGGT ATGTCGTCAG AACACTTTGG CCATAAACTA 23100

CTCAGCCCCA CTAGTGTCAT TGCAAGACAA TCTTACTTTA AGTTATGCTC AACCATTAAC 23160
TGTAAGCGAT AATTCTTTAA GATTGTCTCT AAATTCTCCA CTAAACACAA ATAGTGATGG 23220
AAAACTTAGT GTAAACTATT CTAATCCTTT AGTTGTGACT GACTCTAATC TTACCCTCAG 23280
TGTTAAAAAA CCTGTAATGA TTAACAACAC AGGTAATGTT GACTTAAGCT TTACAGCTCC 23340
CATAAAATTA AATGATGCAG AACAGTTGAC TTTAGAAACC ACTGAGCCCT TGGAAGTGGC 23400
CGATAACGCT CTAAAACTGA AACTTGGAAA AGGCTTAACT GTTAGTAATA ATGCTTTAAC 23460
CTTAAACCTT GGAAACGGTT TGACTTTCCA ACAAGGTCTT TTACAAATTA AAACTAATAG 23520
CTCTCTAGGG TTTAATGCTT CTGGGGAATT ATCAACAGCT ACAAAGCAGG GAACCATAAC 23580
CGTTAACTTT CTAAGCACAA CTCCTATAGC TTTTGGGTGG CAAATAATAC CTACTACTGT 23640
AGCTTTCATT TATATTTTAT CAGGAACACA ATTTACTCCT CAATCCCCAG TAACTTCTTT 23700
AGGTTTTCAA CCCCCACAAG ACTTTTTGGA TTTCTTCGTT TTAAGTCCGT TTGTTACATC 23760
TGTAACTCAA ATTGTGGGAA ATGATGTTAA GGTTATTGGC CTAACTATTT CTAAAAACCA 23820
ATCTACCATA ACTATGAAAT TTACTTCTCC CTTAGCTGAA AATGTACCAG TTAGTATGTT 23880
TACAGCACAT CAATTCAGAC AATGAATATT TTAAAAATTC TTTATTAAAG AGTAATCTTT 23940
TTACATACCG TTCTTGACAT AATGTGCCTC TATAATTAAC AAATCTAAGC AAGCAAGGTT 24000
GATCATTGGA ATCTATAGAA GCATAACTCT TCCAATAAGC ATAATCATAT GGCGGTAAAT 24060
GAAAACCCCT TAAATCTACC ATATTCATCT TTAAGTGTAC AGTATCTAAC AGGTTTTTAC 24120
AATCTTGCAC TTCTGGACTT TTAAAAACAA ACAGTACTTT CATAGGACAA CAATTGTAAC 24180
GGTTATAATC TGTTACAATT TTACTTATTT CTTCTTCCAA TGGCAAAGCA TTCCAAAGTC 24240
TTGTTATAAG TACTGTAAAA TCATCAAATG AATAACATAA CACATTTGTA CAACAATTGG 24300
TCCAAGGTAA AAAAACAGGC ACACGAACAT GAACTTTTTT TAAAATTAAC ATCAGTGTCT 24360
GTTTTAAACT TTGACATTGC AAAGAATTTG GCTGCAAGCA ATGACAATGA AATTGATTTT 24420
GCTGACAAGG TAAGTCACAC AAATACAACT TTAACAGCCT AAATATAACA ACATTAATGT 24480
AACTTTCCAA GACTTTAAAA CTAACAAACG GTATATCACA ATAAAAAAGA TGATGAATCC 24540
CTTCGCAACA CATAATGGAG TTCATGCTAC ATCCAAAGAT GGTTCCGACA AACCTCTGTA 24600
AATTAAAGAA CAACAATACA ACATACGAAG AAAATTAAAA CGTTTTTCAA AACGAGATAT 24660
ACATTGCTGC AAAGTATCTG AACATTTACA TTTTATACTT ATAAGCTCAC AAGTTTCAGA 24720
AAATGTAATT CGTTTAACAG TTTGATATGA ATACCATTTT GAAGAAAAAT AGAAATAGTT 24780

TTGTGCATTT GTAAGCTCCC AGAAACATTA ACGGACAGGC AAATCCAAGT ATTACAACAA 24840

ACAGGAACAG TCTTAACGTT TCGTTCAGAA AACAAAGTAA CAGGCATATG ATTAAAGCAA 24900

GACAATAAAA CACTTTTGGC AGCTAAACAT TGCAAAGATC CAGGTGAATT ACAATGACAA 24960

TGATAATAAA ACTTATAAGC CATATCGGCC CTCTTGCAAA ACGAATCAGC TTTTTGGCTT 25020

ATAGGAAAAT AACAAAAAAA CTGATTATAT ATGAATGGAG TTAATATCTT CTTCAAATTA 25080

TACACACGAA TAGCAGAACC AAGACGACCA CGCCCAACAC AGGTAAATAT TTCAAGTCCA 25140

TGACTAGGAA CAGATGGTTT CTCACAAGCA ACAACTTTGA TTTGCTTATC CATCACTGCC 25200

AATCAGGCTT AATAGGAAAA GAAGAAAAAT AATTTTCCCA ATAATAACGA AAGAAATTCC 25260

ACGTTTCATC CTGTACATTA CTAGTCACAA ATACAACCTC CGCTATCAAA GATTCCCTAT 25320

CATTTAAAAC TCCCACCAAA TTGTCCCAGT CTACCTCAAA AAAGCCAGTT CCCATATTTT 25380

CAAAATTTGC CCATTTTAAA TAATCCAAAG CATCAAATTC AGGAAACAAA TCTTTCTGAG 25440

CTAAAACATA TACAGTTTTA TCGCCATTAA ATCTAAAAGC CATCCTAAAT GGACCTCTAG 25500

CCCAGTAGTT TAAGTACCGG GAAGAGACTA TACAATATAC TTGATATTGA TGTCTGTTAA 25560

GTGGTGATAA AAAAGAAAGT AATTCAGAAT TAGGATAAAG CATTCTCCCA TGTTGATTCA 25620

TCTACAAAAA ACAAAAAAAT TATAAGGTTC ATAGAAAACC TACTATTTAA CAAATCTATA 25680

AAAATGCATT AAAAAGTTAC CTTGAATATA AATTCAGATC ACCTAAAAAA CGAAAAAAAA 25740

TAACATTTAT GTTAGTAAAT GATAGTCTTT AAAAATTAGA AAAGAATCAA GTCGCTTTTA 25800

TACTTACAAA CTCCAAATAA ATTCTGTAAC CAAGAGAAAA ATTGTAACCT AAAAGGTAAA 25860

GAAGAACATT ATAAGATTAA AACCACTCTA AAATCTGAAA AGCATTATGA AAAATTCTGA 25920

TAGCTGCAAC TTACTAGTCT TCTCCAAATG TTGCAGGCAT TTCAAAAAAT CAAGAGGAAA 25980

ACCGGAGTTT ATAAAGTAGT AGTCTGATTA TATCTGAAAA AGTTTAACTT CCTTTTCAAC 26040

CCAACCCAGT CCAATAAAAT TCCAACCTTA ACTTCTTTCC TGCTAAAACT CCATAAAAGT 26100

CCAATTACCA CTTGACTTTT ATTTAACCTC AATTATGTTA CATGTTATTC TACCCATAAA 26160

AACTTGATGA CCAAGAACTG ACCTTTCCCA TGTTTTTCTG AAATAACAAA AATGTTGATT 26220

TAAAGATTTT TAACTACCCA AAAAACCCGC TCTCATGATT TTTTCTTATA TAAACAGGAT 26280

ACAAAAGAAC TGGCAAAGAT ATTCCATCAT ACTTCTCCAA CTGTCAAAAC ATACCACTTA 26340

ACCTCTCCCA TGTTTTTTCC CTTTTGCACA AACAGGATAT AAAAAATATT TTTGCCACAA 26400

TGTTTTTCCT TTTACTCAAC TGCCAGAATA AAAATGAACA GCTTAACCTT TTTCCCTCTT 26460

AACCCATTGC GTTCCTCTAA GAAAAAAATT ATCCCGCCCA ATATGCTAAA GGCTTCTCCC 26520

GCCAAAACAG CTCAACTTAA AATCTCTCAT GAATAAAACC CAGAGAAAAT TTCCAGTAAT 26580

AAAAATTAAT AACCGTGAAG TACTAGATCT AATAATGATA TTTTGAACTC ATAAAAATCC 26640

ACCATCCATG TAATGTTACA AACACTTTTT TATTGAGTTT TTTCTTACAA CTGCATTACA 26700

TACAGGCCAA GCATCAAACT TTCTTCTGTA TTTCTTCCTA GACCACAAAA TTACAGACTT 26760

ATATTTCTGC CACAAATCTC TATGATCTTT ACAGTAACAC TTACATTTAA ATGGGGAATA 26820

CAGCAGCAAA TAAGGATGAG TTAAACATGC GATACAATGA CCAGAAGGAA GATAATACAA 26880

TACATCACAC CAAAATGAAG GTACAGACAA CATCGCATGA AATCTTAAAT GTGATTTTAC 26940

AATAAATTTC TGCAGCAGCT TACAATCTAT ATTAGCAAAC CGTTTTATAT ACAAACATAA 27000

AAACTTGGAA CTTTTCACCA ACTCAATCAT GTTATTATAA CACATTACAA ATTTTGCTAT 27060

ATCTTTATTT GTCAAATAAC AAAATATCTC AATCCACAGC TCATCTGGCA GCAAACTTCG 27120

CAAATCCATG ACCTGTAAAA GATACAACAG AAAACAGAAA ATTAATGCCA TTCAATAACA 27180

TAAAAAATAC AGTCAAATCA CATACTTTTT CTCACTTACA AAACTTTGTG AGCAGGCCTC 27240

CAAAACAAAC TTCAGAAAAT GGATGCATAC AAGAACATTC TCCTCTCAAA AATTGCTTTA 27300

ACTGAATGCG GCATTTTGCA CCTCCAGAAA AATGCAGTCC ATTGAGAGGC TCTTCTCTTA 27360

AAACACAGAA ATGCTTCTGC AAAATCTGTA AAGAAACTAA CAACTTCCAA ATTCCAATCA 27420

TCATGCATTG CAAAGAAGGA CATTCAACAG CAAAAGGATC GTGATGAGCC AATAAAGCTT 27480

TACTGTATGA CTCATTTTCA TGAATTACAG TCTGTAACTT ACTATAATGC ATTTTAAGCT 27540

CTGCTTCACA AATTAATAAT GCTAATTTCT TTAAGCAGCT CAAAGAAAAC TCATCAGGAC 27600

AACGGCATTT AAGAAAGCAA CAAAATGATT TCTTAAAATA CATTTTTCCA GCATGATGAA 27660

CAATAAAAAA TTTCAACGTT AAACAATGCA AAAATGCATT TTTATGCACA GTGAAAGTAA 27720

TTTTTTCAGC TGAAGCTAAA TCACAGCCTA TTTTATTACA TGATTTTGTA TGCTCCAAAA 27780

GAGCTTGTTT TAATTGCTTC AAATCCATCT TCTTACAATT TTTTCTTTTT ATAAACACCA 27840

GAACCGCATT CAGGCCAATT CCAGTTATTG TTTAAATTTG CTACAGAAAC TGCAGACCAC 27900

AAAACCACAT CCTCTAAATC AACCCACAAA GATCTATGAT CCACACAAAA ACACAAAGAA 27960

TGATACGGAG AATACAACAA TAAATGGGGA TTAACAAGGG ACGCAACACA ATGACCCGAA 28020

GGTAATAAAG TTTTACAGCA CCAATTACAA GCAACAGGTA ATGGAGTATA TTTCCCAATG 28080

CGACGAGAAA GCCGAATGTC ATTCAGAACA GCATTGCATT TTATCTTCTC AAACCTCTTA 28140
AGGTGCAATT GTATAAAATA AGAATCCTTA ATGACAGTGA TGAATTGAGG AAAAGCAAAA 28200
ACAAAACTAG CAATGTCTTT GCTTGTAAGT TTCAAAAATA TCTTCATCCA AATCTCAGTC 28260
GGTAATTCAA CAAAAAATTC AGGGGCCTAC AAAATTAATC AGACTAATTT AATATCATCT 28320
TGTAAACAGC GAAAAGAAAA AATAACACAC CCAAAAATAA AAAACTCTTA CCCCTGTTAT 28380
CCATCGAGAT ACACAGAAAA ATTCAGAACA CTCAGTGTCA TGTTTCTTAA ATTGTTCCCA 28440
AAGCTCAGAC ATTCTAAGCC AAAAATTTTT TGAGAACTGC AAAAACCCAG TTTTTATAAC 28500
AAAGCCTTAA TGTTTTCTTA ACTGATTTAA CTGCCCTAAC AGGAACTCCA CATTCCGGCC 28560
ACCGCCACCC AGGGGACAAA TCTTGCCAAG AACTACAAGT CCATAAAACA ACATCCTGCA 28620
AATTATACCA AAGGTTTCTA TGGTCGACAC AATTACAACC TGACCTAAAA GGTGAATAAA 28680
GCAGTAAATA AGGATGAGTT AAACAGGCCA CACAATGTCC AGAATGTAAA AAATGCTTTG 28740
TTTGGCACCA ACCAGACCAC AGCTGAAGCA AAGGAAAATT GTAGCGAACA CATTCTTCTC 28800
GTAATCTGTT TAACACAGAA CAACATTCAA TTCTGGCAAA CCTCTTTAAA AAATGTTTTC 28860
TGAAATATTT CTTTAAAATG ACAGTTTGCA ACTCTGGAAA ACACAAAATA AAAGCCGCAA 28920
TATCTCTACT GCTTAAATAT AAAAATATCA TTGTCCAAAT TTCTACTGGT AAAACTGAAA 28980
GCATCTTCTT CCTATTAAAA AAAGAAAAGT GTTTTCAAAT TATATTAGAC TCTAACCAAA 29040
AAAATTCAAA TACTTTTCCT TTATAATGTA CATTAAGAAT AAAAATATAC TCACCGTTTA 29100
AAAGTAGAAC TTAACAGTAT AATATAAATA CAAGTGAGCT GAACAACGAC AGCCGATTTC 29160
AGCCGGAGCA AAATTAAAAA GAATAAAAGG ATCAAACCAA CACGTAGGAC AGTCTACTCC 29220
AAAACAGTAA CGGCAGTATG ACACAGAAGG AGAGGAACTA AGTCCAGGAA ACTTCGCCCG 29280
GTGCGATAAA AAGTAACGCC GCCGGAAAGC AGTTGAATAC AAAAGAGGTA AAAATTCACG 29340
AAAAACAGAA GCAAAAACTA CTAAATCTGC TATTGGCAAA TAAAGAAAAA TTTCAAACCA 29400
TATTTCCAAA GGAAGAAAAG CAATCATACC GTAGAAGAAC CTGAAGGCGA CCGCAAACGT 29460
GCTCCCGTAC CACAACGTCA CACGCCACAC CCACTGGGAA AACCCACACG CCCCGCCTCT 29520
GTGCAACGTT ATATATATGA ATAG 29544

## Description

### Technical Field

The present invention relates to new viral vectors derived from a benign adenovirus (OAV287) isolated from sheep in Australia and also discloses the use of these vectors for the delivery and expression of nucleic acid sequences encoding functional RNA molecules or polypeptides to animals.

### Background of the Invention

Diseases caused by infectious agents and parasite infestations cause health problems and production losses in domestic animals but for many infectious agents no vaccine exists. Consequently, there are major research efforts worldwide to develop new vaccines which can protect against disease.

While some protective antigens from infectious agents and parasites have been identified, their successful use as vaccines requires the development of systems which can effectively deliver the antigen to the host. A variety of recombinant gene expression vectors derived principally from the pox virus family have been employed as these are generally of low pathogenicity. Expression of the foreign protein following infection by the recombinant viral vector may stimulate a protective immune response in the host.

However, no viral vector has all the attributes desirable for all situations. Some vectors are better suited to particular tasks than others because of their biological properties. For example, it has often proved difficult to stimulate an effective mucosal immune response which can protect against disease. In humans, adenoviruses have been given orally to vaccinate against respiratory disease (1). As this involves protection at mucosal surfaces adenoviruses clearly have potential in this regard. Human adenovirus vectors have also been used to deliver genes to muscle (2) and other tissues. Although adenoviruses do not generally integrate their DNA into the cellular genome, nevertheless, the DNA persists and long term protein expression is observed. Expression of an appropriate antigen from such cells can generate a systemic immune response which may be protective against the homologous disease causing agent.

Known adenovirus genomes are linear double-stranded DNA molecules which have an inverted terminal repeat sequence (ITR) at each end and a protein covalently bound to the 5'-terminal C residue (3). The genome sequence and structure has now been completely determined for human adenoviruses types 2, 5, 12 and 40 and partially for numerous others, including some animal isolates (see Genebank and EMBL Nucleic Acid databases). Human adenovirus type 2 was the first genome to be sequenced but broadly speaking its genome arrangement is conserved among other characterized adenoviruses i.e. early regions E1-E4 and the structural protein homologues can be recognized in similar locations in the genome. In particular, the E1A/E1B region is located at the left hand end of the genome and region E4 is always located at the right hand end of the genome. Early region E3 is always located between the genes for structural proteins pVIII and fiber, although its size and complexity varies between species e.g. from 3kb with at least 10 open reading frames in human adenoviruses to approximately 0.7kb with only two significant open reading frames in murine adenovirus (4, 5). E3 is a key region for the construction of recombinant viruses as it is non-essential for replication in vitro (6). The late, L region is expressed from the major late promoter, MLP and complex splicing generates families of mRNAs which code for most of the structural viral proteins. Proteins IVa2 and IX appear to have their own promoters.

Although there are some human viral vectors available for medical use there are few animal viral vectors suitable for use in veterinary applications. In order to obtain a more suitable animal viral vector the present inventors have purified an ovine adenovirus (OAV287) isolated from sheep in Western Australia. This ovine adenovirus is serologically related to bovine adenovirus type 7 but is genetically distinct from the bovine adenoviruses and other Australian ovine isolates, as shown by comparisons between the ovine and bovine adenoviruses, based on restriction enzyme profiles (8), The genome arrangement of the virus according to the present invention varies significantly from all other known adenoviruses. The adenoviral DNA molecule of the present invention is suitable for use in viral vectors capable of expressing a variety of polypeptides when used for veterinary applications.

### Summary of the invention

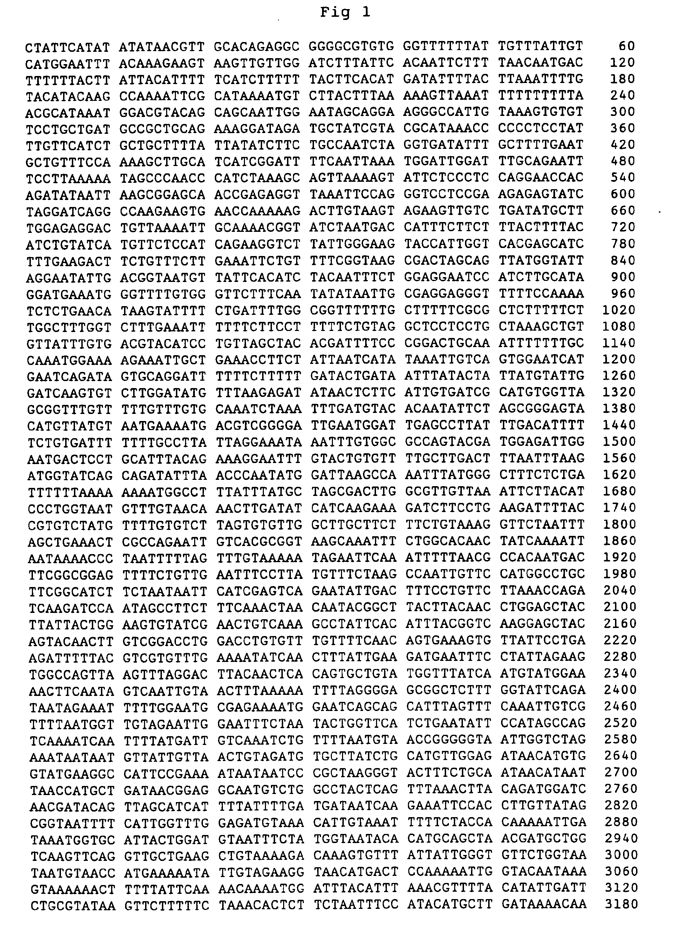

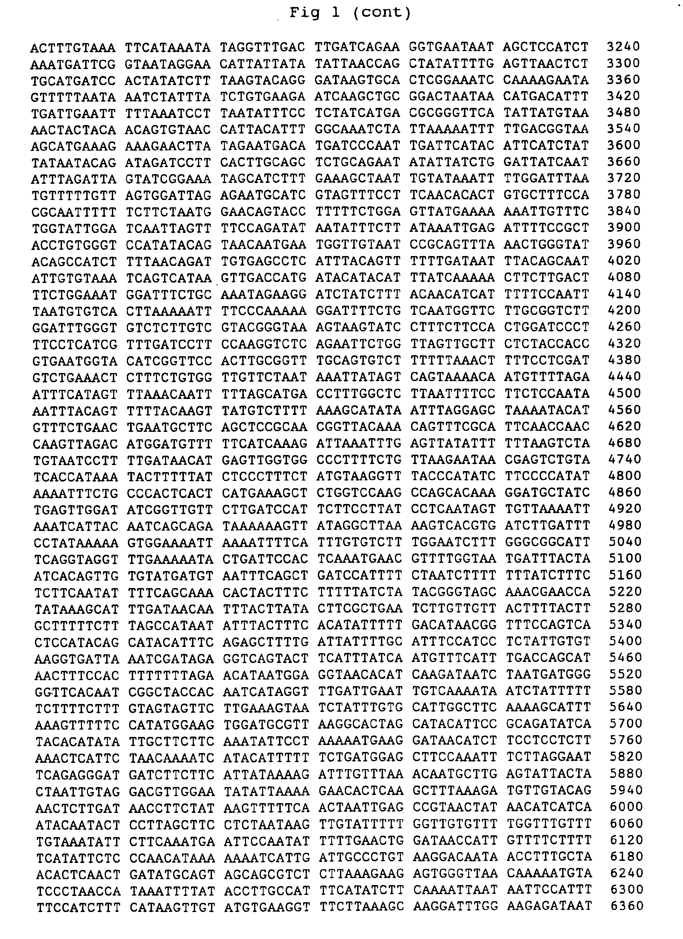

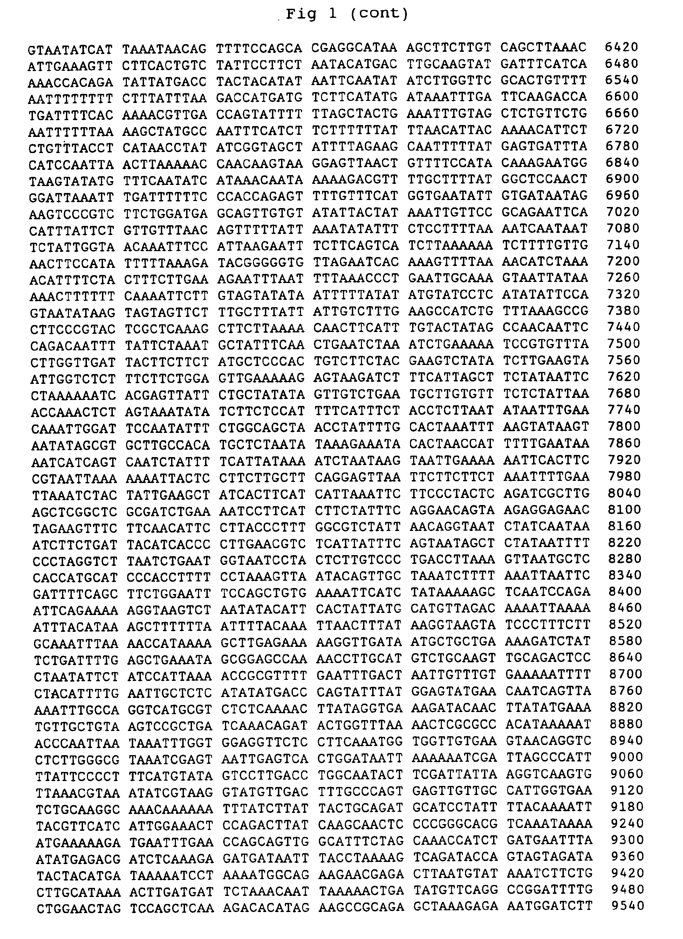

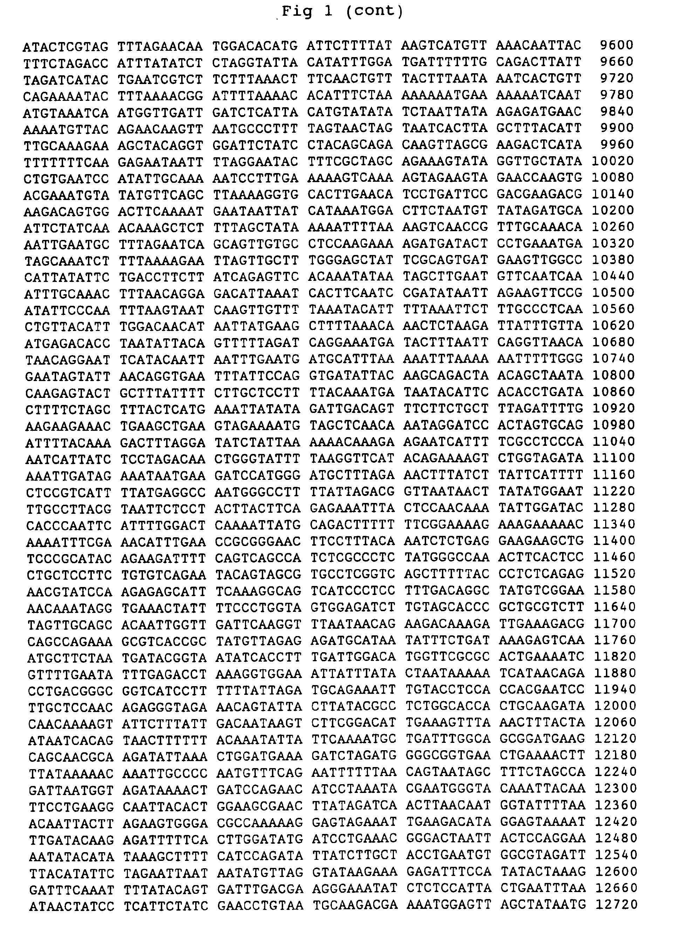

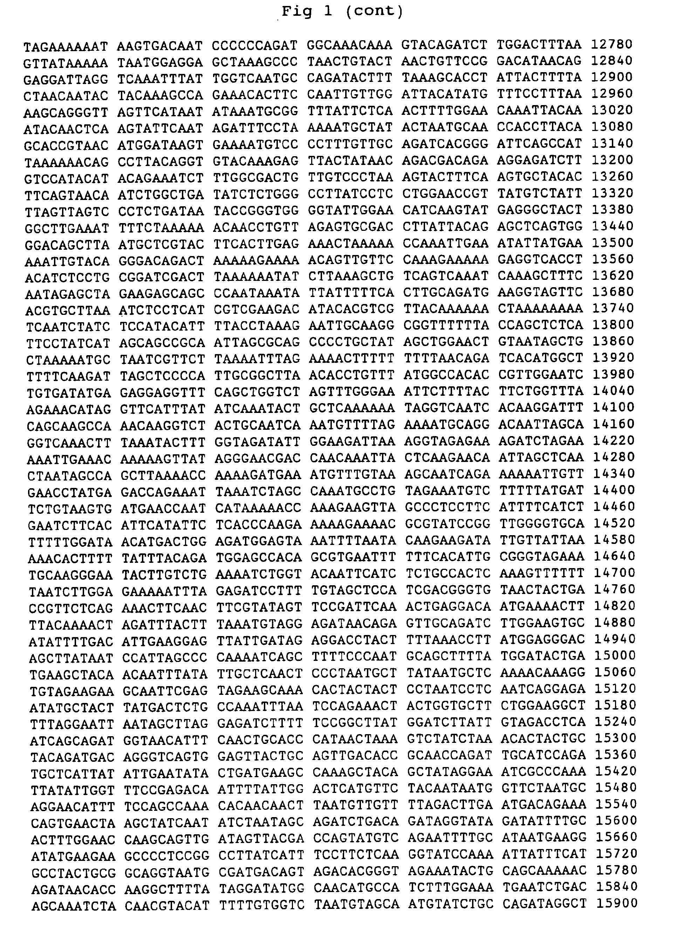

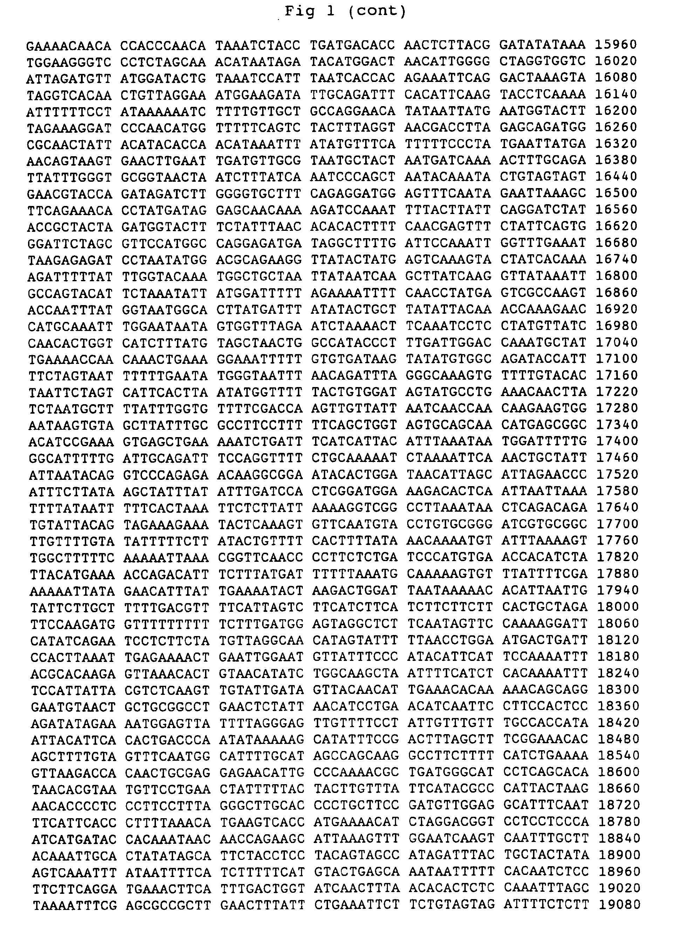

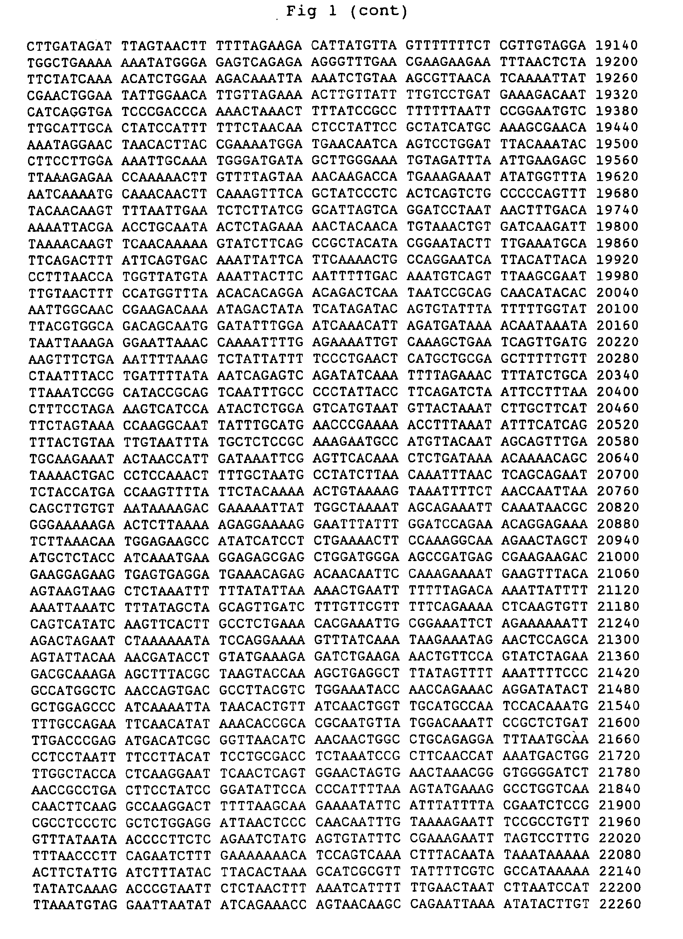

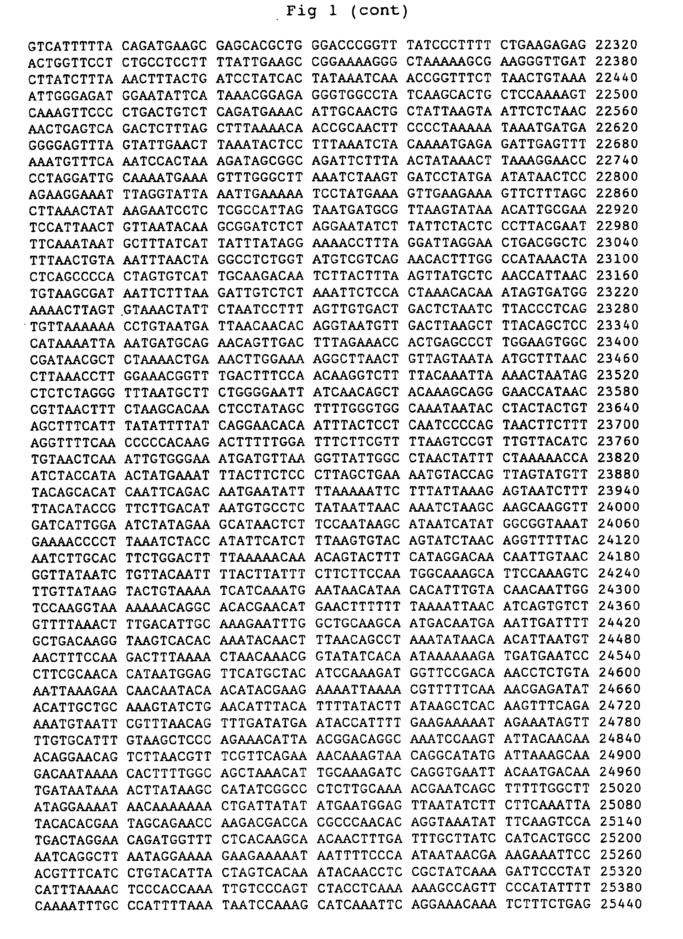

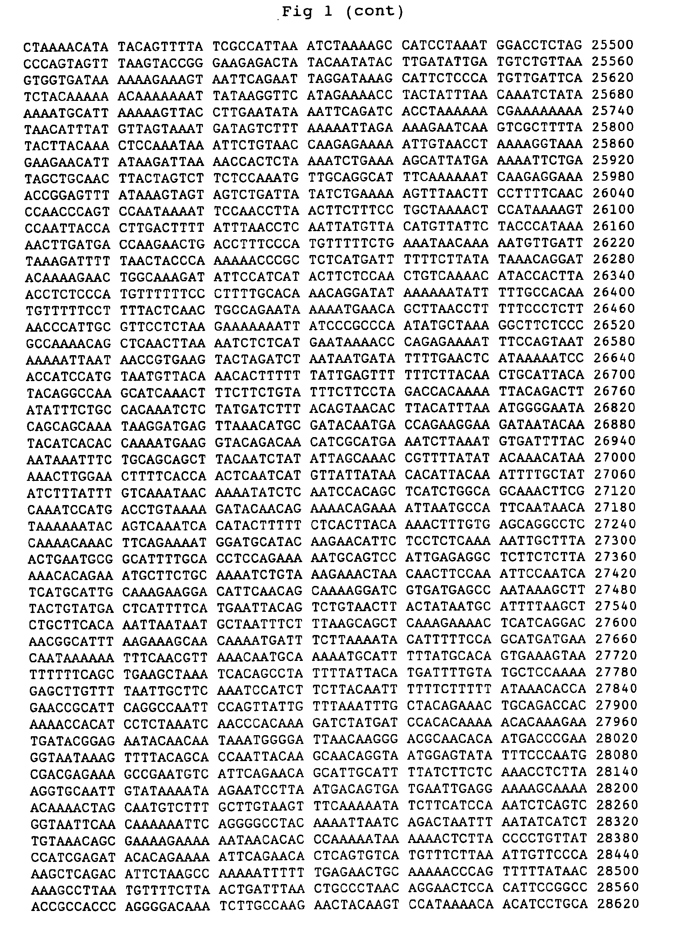

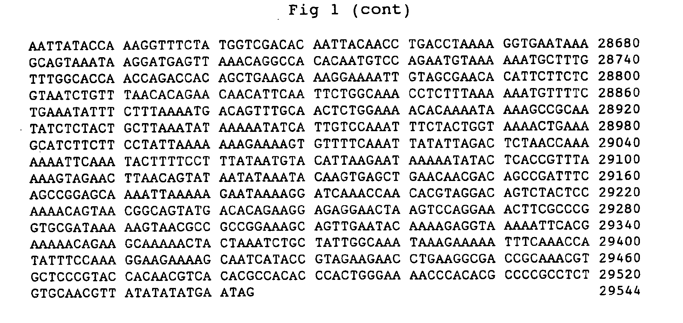

According to the first aspect, the present invention provides a recombinant ovine adenovirus comprising at least one nucleic acid sequence encoding a non-adenoviral polypeptide, wherein the recombinant adenovirus comprises the nucleic acid sequence as shown in Figure 1. The nucleic acid sequence encoding the non-adenoviral polypeptide may encode a polypeptide derived from bacteria, viruses, parasites or eukaryotes. The non-adenoviral polypeptide may be rotavirus VP7sc antigen or *Trichostrongylus colubriformis* 17 kD antigen. The functional element of the ovine adenovirus may be selected from the group consisting of promoter, gene, inverted terminal repeat, viral packaging signal and RNA processing signal.

In a second aspect, the present invention provides a method of delivering a nucleic acid encoding a non-adenoviral polypeptide to a target cell *in vitro,* the method comprising infecting the target cell with the recombinant ovine adenovirus of the first aspect of the present invention such that the nucleic acid sequence encoding the non-adenoviral polypeptide is expressed by the target cell.

In a third aspect, the present invention provides the recombinant ovine adenovirus of the first aspect of the present invention for use in delivery of the non-adenoviral polypeptide to an animal. The animal may be a grazing animal, preferably a sheep.

In a fourth aspect, the present invention provides a recombinant ovine adenovirus (OAV287) of the first aspect of the present invention comprising at least one nucleic acid sequence encoding a functional RNA molecule. The functional RNA molecule may be an antisense RNA molecule, a ribozyme or a messenger RNA molecule.

In a fifth aspect, the present invention provides a method of delivering a functional RNA molecule to a target cell *in vitro,* the method comprising infecting the target cell with a recombinant ovine adenovirus of the fourth aspect of the present invention such that the functional RNA molecule is expressed by the target cell.

In a sixth aspect, the present invention provides the recombinant ovine adenovirus of the fourth aspect of the present invention for use in delivery of the functional RNA molecule to a target cell.

In a seventh aspect, the present invention provides an isolated DNA molecule derived from an ovine adenovirus of the first aspect of the present invention wherein a non-essential region is deleted or altered.

In an eighth aspect, the present invention provides an isolated DNA molecule derived from an ovine adenovirus of the first aspect of the present invention, wherein the DNA molecule comprises at least one nucleic acid sequence encoding a non-adenoviral polypeptide, or at least one functional RNA molecule, inserted in a region of the DNA molecule corresponding to the SalI site between nucleotides 28,643 and 28,648 as shown in Figure 1.

In a ninth aspect, the present invention provides an isolated DNA comprising an inverted terminal repeat having the nucleic acid base sequence of 1 to 46 as shown in Figure 1.

In a tenth aspect, the present invention provides a plasmid comprising the DNA molecule of the seventh, eighth or ninth aspects of the present invention. Preferably, the plasmid further comprises a nucleic acid sequence encoding an origin of replication and a further nucleic acid sequence encoding a marker. Preferably the marker encodes for resistance to an antimicrobial agent. More preferably the antimicrobial agent is ampicillin. Sequences encoding inverted terminal repeats of the adenovirus may be joined.

In an eleventh aspect, the present invention provides the recombinant ovine adenovirus of the first aspect of the invention for use in delivering a nucleic acid encoding a non-adenoviral polypeptide to a target cell.

The invention encompasses functionally equivalent nucleic acid sequences to OAV287. As used herein the term "functionally equivalent nucleic acid sequence" is intended to cover minor variations in the ovine adenovirus (OAV287) DNA molecule which, due to degeneracy in the DNA code, does not result in the molecule encoding different viral polypeptides. Further, this term is intended to cover alterations in the DNA code which lead to changes in the encoded polypeptides, but in which such changes do not substantially affect the biological activities of these viral polypeptides.

As used herein the term "functional element" is intended to cover nucleic acid sequences that encode promoters, genes, inverted terminal repeats, viral packaging signals and RNA processing signals. It will be appreciated by one skilled in the art that unique sequences from ovine adenovirus (OAV287) that encode these functional elements may be useful in other systems including plasmids and non-ovine adenoviral vectors.

In order that the nature of the present invention may be more clearly understood preferred forms thereof will be described with reference to the following examples and the accompanying drawings.

### Brief Description of the Drawings

Figure 1 is the nucleic acid sequence of the OAV287 genome beginning at base 1 of the left-hand ITR.
Figure 2 shows the arrangement of OAV287 genes based on homologies detected with Ad2. Regions with question marks are tentative identifications because of the lack of obvious homology.
Figure 3 indicates the major open reading frames in the proposed E1 region of OAV287. Asterisks show the location of possible initiation codons. A previously unidentified gene (p28kD) which codes for a processed structural protein is encoded on the complementary strand.
Figure 4 shows open reading frames in the region of the OAV287 expected to contain E3. However, E3 is missing as the gap between the pVIII and fiber genes is only 197 nucleotides. The site at which the ApaI/NotI polylinker was later inserted is indicated.
Figure 5 shows the major open reading frames in the probable E3 region of OAV287. Asterisks show the location of potential initiation codons. The SalI site which was modified by end-filling and re-ligation and the alternative site at which a polylinker sequence was later inserted into the genome without loss of infectivity is indicated.
Figure 6 is a scheme describing the construction of a plasmid (pOAV287Cm) containing a full-length clone of the OAV287 genome with pACYC184 sequences inserted in the SalI site. Filled in regions show OAV287 sequences. Cross-hatched sequences are derived from plasmids pUC13 or Bluescribe M13+ (Amp^{R}), stippled regions from pSELECT (Tet^{R}) and open regions from pACYC184(Cm^{R}). Only the key restriction sites used for plasmid construction are indicated.
Figure 7 shows a map of the plasmids pOAV100, pOAV200, pOAV600 and pOAV600S. Arrowheads indicate the ITRs and the approximate location of the major late promoter (MLP). The mutated SalI site and sites at which the ApaI/NotI polylinker sequences were inserted are indicated. Light hatching signifies modified Bluescribe sequences inserted in the KpnI site. Linear, infectious genomes (dark hatching) are released by digestion with KpnI.
Figure 8 shows the results of screening ovine adenoviruses OAV100 and OAV200 rescued by transfection of recombinant plasmids pOAV100 and pOAV200 into CSL503 cells. Portions of the genome spanning (A) the mutated SphI site in OAV100 and (B) the ApaI/EcoRV/NotI polylinker insertion site in OAV200 were amplified by PCR together with the corresponding regions from wild-type OAV287. The products were digested with SphI (A, lanes 3 & 5) and ApaI, EcoRV or NotI (B, lanes 3-5, and 8-10, respectively). (U) indicates undigested samples.
Figure 9 is a map of a plasmid pMT used for the assembly of gene expression cassettes. Fragments containing the OAV287 major late promoter and tripartite leader sequences are linked and precede a multiple cloning site for the insertion of genes of interest. A tandem polyadenylation signal (AATAAA) follows.
Figure 10 shows a summary of recombinant viruses which have been rescued from the corresponding infectious plasmids and the gene expression cassettes they carry. Cassettes were inserted into the OAV genome between the pVIII and fibre genes as indicated.
Figure 11 shows the expression of (A) the *T. ovis* 45W and *L. cuprina* PM95 antigens in CSL503 cells following infection of these cells with OAV205 and OAV210 viruses, respectively and (B) VP7sc expression in CSL503 and bovine nasal turbinate cells following infection with virus OAV204. (I) Infected cells (U) Uninfected cells. (M) indicates marker proteins of the sizes shown.
Figure 12 shows expression of VP7sc in (A) CSL503 cells and (B) rabbit kidney and bovine nasal turbinate cells following infection with OAV206 virus. (I) Infected cells. (U) uninfected cells. (M) indicates marker proteins of the sizes shown.

### Description of the Invention

### METHODS

### Growth and Purification of OAV287

The virus, isolated from sheep in 1985, was obtained from R.L. Peet, Animal Health Laboratory, Department of Agriculture, Western Australia. The virus isolate was grown in sheep foetal lung cells (line CSL503) and twice plaque-purified under solid overlay before stocks were prepared. Virus was purified from CSL503 cells as described previously (18, 22). DNA was extracted from the virus by digestion with proteinase K (23). Cloning of Genome Fragments

Molecular techniques for manipulation, modification and transformation of plasmid DNA which were used in the work described below are described in (9) and similar publications. OAV287 DNA was digested with various restriction endonucleases including BamHI, SphI, SmaI and SalI to deduce the location of these sites (18).

The adenovirus genome has a protein covalently linked to each end of the linear dsDNA (24). The BamHI A and D fragments of approximately 8kb and 4kb, respectively, were identified as the terminal genomic fragments because their migration into agarose gels was dependent on the pre-digestion of viral DNA with proteinase K. The internal BamHI fragments B, C, E and F, estimated at 6.2, 5.1, 3.4 and 1.1kb in size respectively, were separated on an agarose gel, recovered and cloned into BamHI-digested pUC13 using standard ligation and transformation procedures (9). To clone the terminal BamHI A and D fragments, viral DNA (10µg) was digested with proteinase K (50µg/ml in 10mM Tris/HCL, pH8.0, containing 1mM EDTA and 0.5% SDS) at 65°C for 60min to remove the terminal protein. The DNA was extracted twice with phenol/chloroform, once with ether and recovered by ethanol precipitation. The 3'ends (of unknown sequence) were then digested exo-nucleolytically with T₄ DNA polymerase (5 units, Toyobo, Tokyo, Japan) in the presence of dATP (100µM) in buffer containing Tris HCL (50mM), pH8.0, MgCl₂ (7mM), 2-mercaptoethanol (7mM) and BSA (10µg/ml) for 15min at 37°C. The DNA was again purified by phenol extraction and ethanol precipitation described above. To remove the single-stranded terminal regions and create blunt ends the DNA was digested with 1 unit of mung bean nuclease (Pharmacia, North Ryde, Australia) for 10 min at 37°C in buffer containing Na acetate (30mM), pH4.6, NaCl (50mM) and ZnCl₂ (1mM) before extraction with phenol/chloroform and recovery by ethanol precipitation. Finally the DNA was digested with BamHI (Pharmacia) and the fragments were separated by electrophoresis in low-melting-point agarose. The BamHI A and D fragments were excised, recovered by NACS column chromatography (BRL, Gaithersburg, Md) and ligated with BamHI/HincII-cut plasmid Bluescribe M13⁺ (Stratagene, La Jolla, Ca) prior to transformation into *E. coli* JM109. Positive clones carrying fragments of the expected size were identified, restriction digested and confirmed as correct by nucleotide sequencing and comparison with partial sequence determined directly from genomic DNA. This revealed that three 3'-terminal nucleotides were removed during the cloning procedure. Nucleotide Sequencing of the OAV287 Genome

The complete sequence of the OAV287 genome was determined by sequencing the BamHI fragments A-F using the Sanger method (25) and various kits provided by commercial suppliers. Nested deletions were constructed for the five largest fragments using a double-stranded nested deletion kit (Pharmacia). These were sequenced using standard primers. Based on newly determined sequence other nucleotide primers were synthesised using a DNA synthesizer (AB1, Model 391). In this way both strands of the entire genome and the junctions between the fragments were sequenced. Mutagenesis of the OAV287 genome

For the construction of a full length OAV287 clone and subsequent modification of it to create plasmids such as pOAV200 and pOAV600 certain mutations were required. A relevant portion of the genome was subcloned into Bluescribe (Stratagene, La Jolla, Ca) or a similar plasmid which allowed rescue of single stranded DNA. Later it became possible to use dsDNA for mutagenesis. Oligonucleotides of the desired sequence were synthesized, phosphorylated and used as primers as described by the manufacturers of Muta-gene Phagemid (Biorad Labs, Ca) or Altered sites II (Promega, Wi) mutagenesis kits. Mutations were generally identified by digestion with the appropriate restriction enzyme or by nucleotide sequencing, or both. Genome fragments containing introduced mutations were subcloned to create larger plasmids such as pOAV200 using appropriate unique restriction sites.

### Construction of a Full-Length Genomic Clone of OAV287

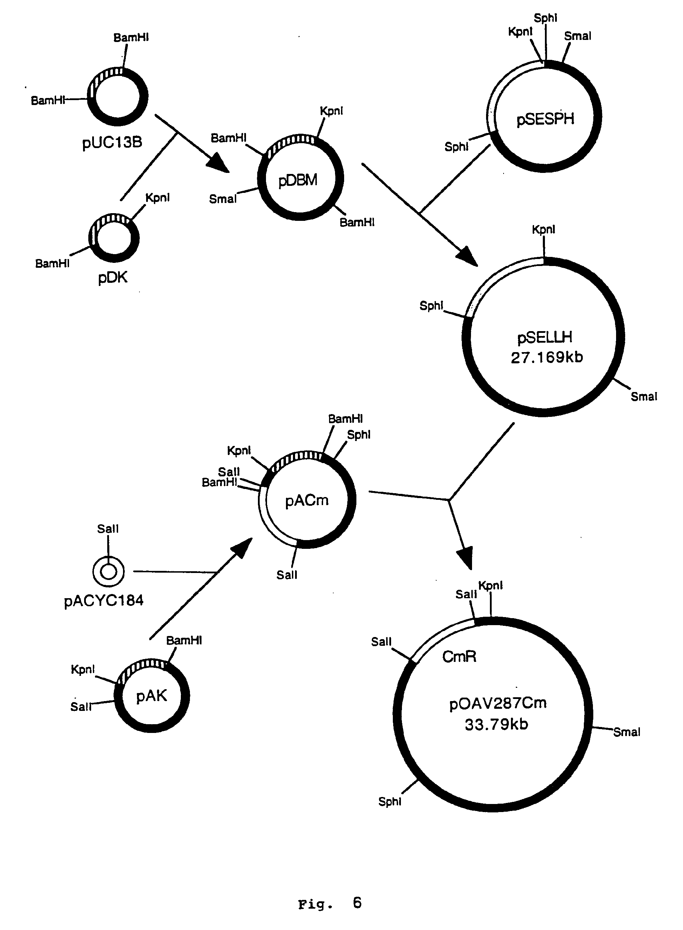

The terminal BamHI A and D fragments (cloned in Bluescribe M13⁺) were each modified by mutagenesis to add the nucleotides lost during cloning and a KpnI site. The last base of the KpnI site incorporated the C at the 5' end of each genomic ITR sequence. This produced plasmids pAK and pDK (Figure 6).

The left hand approximately 21.5kb of the genome was constructed from the BamHI D and B fragments and the SphI A fragment of approximately 13kb. The genomic BamHI B fragment cloned in pUC13 was modified by mutagenesis (GCATGC to GCATCC) to remove the SphI site at position 8287 producing pUC13B. The modified fragment was released by BamHI digestion and cloned into pDK which had been cut with BamHI and dephosphorylated. Colonies carrying the recombinant plasmid pDBM (Figure 6) were identified by screening with an oligonucleotide which spanned the BamHI B/D junction. The SphI A fragment (approximately 13kb) was cloned into the SphI site of pSELECT (Promega) to form pSESPH. This fragment contains a SmaI site near its left hand end which is common to pDBM. The KpnI/SmaI fragment from pDBM was subcloned into pSESPH which had also been cut with KpnI/SmaI to produce pSELLH, a plasmid based on pSELECT which now contained the left-hand approximately 21.5kb of OAV287 DNA.

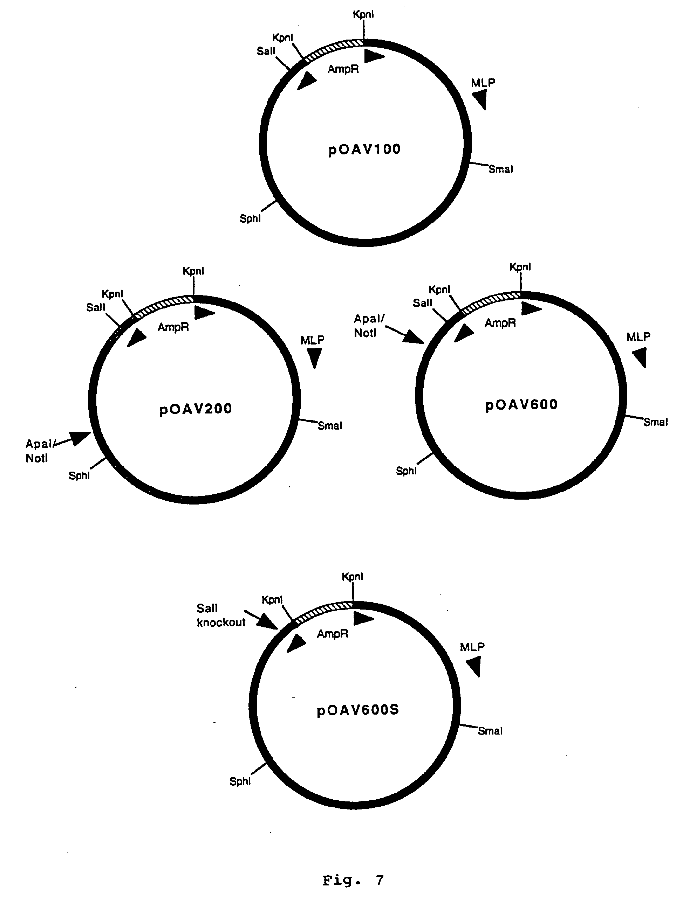

The right-hand end of the genome was constructed from pAK which contains the right-hand approximately 8.6kb of the genome and overlaps the SphI A fragment. pAK was cut with SalI and ligated with SalI-cut pACYC184, a plasmid of 4.24kb which contains a gene encoding chloramphenicol (Cm) resistance and an origin for DNA replication, to form a pACm (Figure 6). This plasmid was cut with SphI and KpnI to produce the right-hand genomic fragment incorporating the pACYC184 sequences. This was ligated with the left-hand KpnI/SphI fragment of approximately 21.5kb prepared from pSELLH to produce the final plasmid pOAV287Cm (Figure 6). This plasmid replicates stably in E. coli and therefore removes the need to propagate the virus to obtain genomic DNA for further study. The recombinant genome in plasmid pOAV287Cm differs from the wild-type viral genome by the single point mutation in the SphI site (base 8287), by the presence of pACYC184 sequences in the SalI site and by the addition of a GTAC sequence between the ITRs. However, insertion of pACYC184 sequences in the SalI site disrupts two significant open reading frames whose functions are unknown. If either of the gene products was essential for replication, then pOAV287Cm could not produce infectious virus following transfection. To circumvent this potential problem pOAV287Cm was modified further. First, plasmid Bluescribe M13- (Stratagene, La Jolla, Ca.) was cut with HindIII and end-filled. The linear plasmid was then cut with SmaI, blunt-end ligated and transformed. The resulting plasmid contained an ampicillin resistance gene and origin of replication and lacked SalI and SphI sites but retained a unique KpnI site. This plasmid was cut with KpnI and ligated with KpnI-cut pOAV287Cm. Plasmids which were doubly resistant to ampicillin and chloramphenicol were selected and grown. One of these was cut with SalI to release the pACYC184 sequences, religated and transformed. The resulting plasmid pOAV100 contained the AmpR gene and replication Ori inserted in the KpnI site between the ITR's of the genome (Figure 7). This plasmid replicated stably in E. coli strain JM109 when maintained in the presence ampicillin (200µg/ml). Large quantities of plasmid were grown for transfection studies. Transfection of DNA and Virus rescue

To determine whether the recombinant genomic clone was infectious, pOAV100 was cut with KpnI to release the linear viral genome and DNA was transfected into CSL503 sheep foetal lung cells using lipofectamine (GibcoBRL). Solution (A) containing plasmid DNA (2-10µg) and 300µl EMEM (containing hepes + glutamine), but lacking foetal calf serum (FCS) and solution (B) containing lipofectamine (10µl) + 300µl EMEM (containing hepes + glutamine), but lacking FCS were combined, mixed gently and incubated for 45 minutes at room temperature. Subconfluent CSL503 cells in a 60mm petri dish were rinsed with 3ml EMEM (plus hepes and glutamine) lacking FCS. EMEM (plus hepes and glutamine) but lacking FCS (2.4ml) was added to the mixture of solutions A and B, mixed gently and added to the rinsed CSL503 cells. Cells were incubated for 5 hours at 37°C in 5% CO₂. The incubation medium was changed using complete EMEM plus FCS (10%) and cells were incubated at 37°C in 5% CO₂ until virus plaques or cytopathic effect was visible (7-15 days).

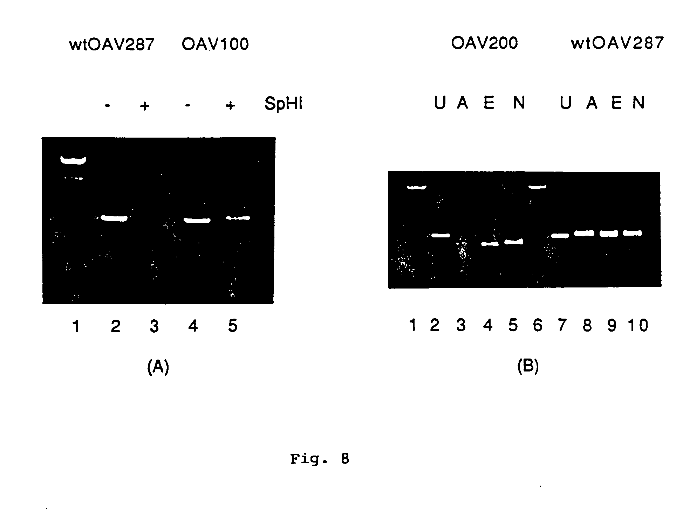

To confirm that viruses rescued from transfection of pOAV100 and pOAV200 were derived from those plasmids a portion of the genome of wild-type OAV287, OAV100 and OAV200 viruses was amplified by PCR. For OAV100 a primer pair spanning the region of the mutated SphI site at bases 8287-8292 was used. For OAV200 the primer pair spanned the insertion site for the ApaI/NotI polylinker between the pVIII and fiber genes. Wild-type OAV287 DNA was amplified as a control in each case. DNA amplified from wild-type OAV287 was cut with SphI whereas the DNA amplified from OAV100 was not (Figure 8A). Similarly OAV200 DNA was cut with ApaI, EcoRV and NotI whereas OAV287 DNA was not (Figure 8B). Other viruses were similarly characterised by restriction enzyme digestion. Identification of MLP/TLS elements and Construction of pMT

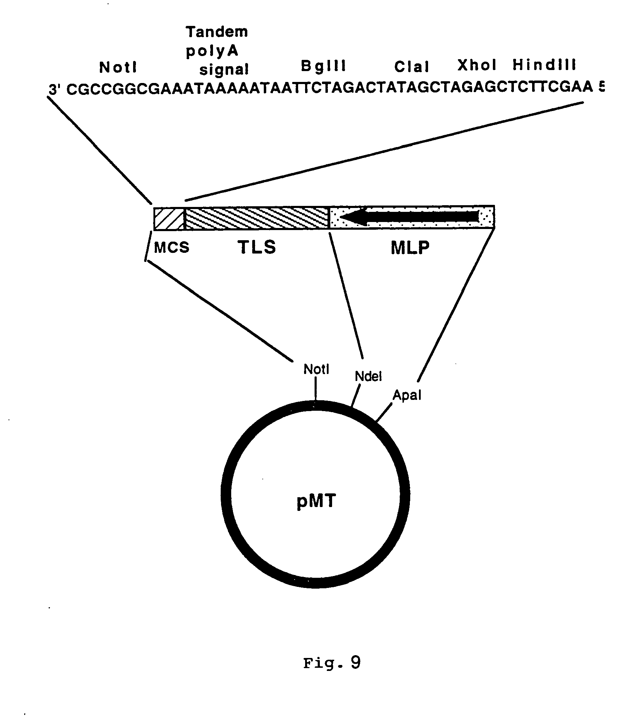

OAV287 TLS elements were identified as follows and as described (17). mRNAs present in OAV287-infected CSL503 cells were copied into cDNA by reverse transcription using primers complementary to the IIIa or fiber genes. A primer thought to fall within TLS exon 1 was then paired with each cDNA primer for PCR. DNA was successfully amplified, cloned and sequenced. This identified TLS exons 2 and 3 (which correspond to bases 8083-8145 and 8350-8412 of Figure 1, respectively) and the 3' boundary of TLS exon 1 which occurs at base 5044 of Figure 1. A second PCR strategy was then used to obtain MLP and TLS fragments suitable for assembly into pMT. The region in Figure 1 between nucleotides 4861 and 5023, thought to contain the MLP was amplified by PCR using a plus sense primer which added an ApaI sequence at the 5' end and a 3' minus sense primer which introduced an NdeI site by point mutation at base 5012. Similarly, the TLS was amplified using a plus sense primer which introduced the NdeI site at base 5012 and a minus sense primer which was complementary to bases 8396-8412 and which added a HindIII site at the 3' end of the PCR product. The PCR fragments were digested with ApaI/NdeI and NdeI/HindIII, respectively and the fragments were cloned into Bluescript SK+ (Stratagene) cut with ApaI/HindIII. The resulting plasmid was then digested with HindIII/NotI and a synthetic oligonucleotide with HindIII/NotI termini and the sequence shown in Figure 9 was cloned to produce plasmid pMT. Genes of interest were then cloned into convenient restriction sites in the NCS. Gene expression cassettes were subcloned as ApaI/NotI fragments into pOAV200 or rescued into infectious virus.

### Infection of cells and expression of antigens

CSL503 and other cells were infected with viruses at a multiplicity of infection of 20pfu/cell as described previously (21). Infection was allowed to proceed for 24-60 hr. Cells were then incubated in methionine-free medium in the presence of ³⁵S-methionine to label newly synthesized proteins. The protein of interest was recovered from cell lysates by immunoprecipitation using a specific antiserum against the expressed protein (21). Recovered proteins were analysed by polyacrylamide gel electrophoresis and detected by autoradiography or using a phophorimager (Molecular Dynamics).

### RESULTS

To characterise the genome in molecular terms, BamHI restriction fragments representing the entire OAV287 genome were cloned into various plasmids and sequenced using methods described in Sambrook (9) and similar publications. Sequences were determined on both strands by using nested sets of deletion mutants together with synthetic oligonucleotide primers which were synthesized from newly determined sequences.

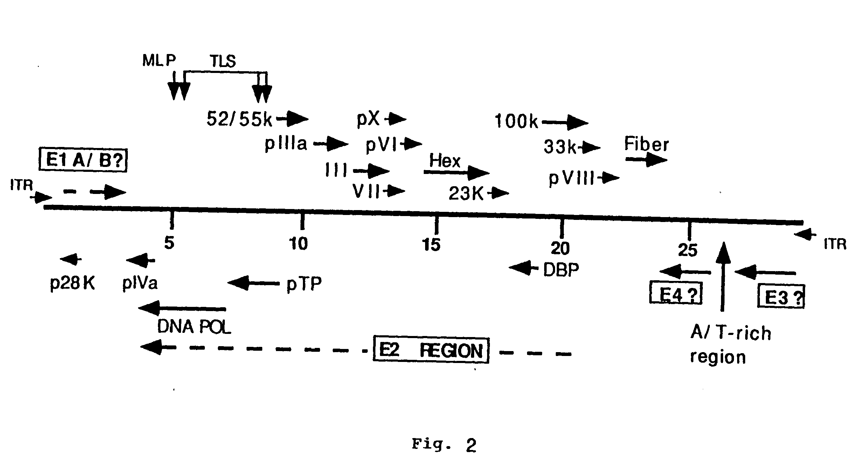

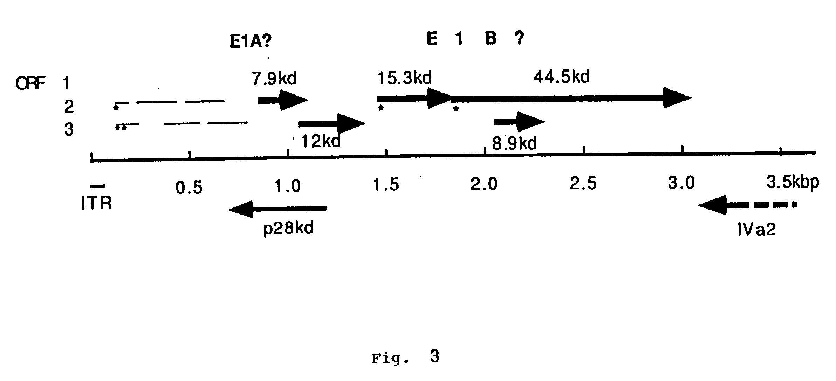

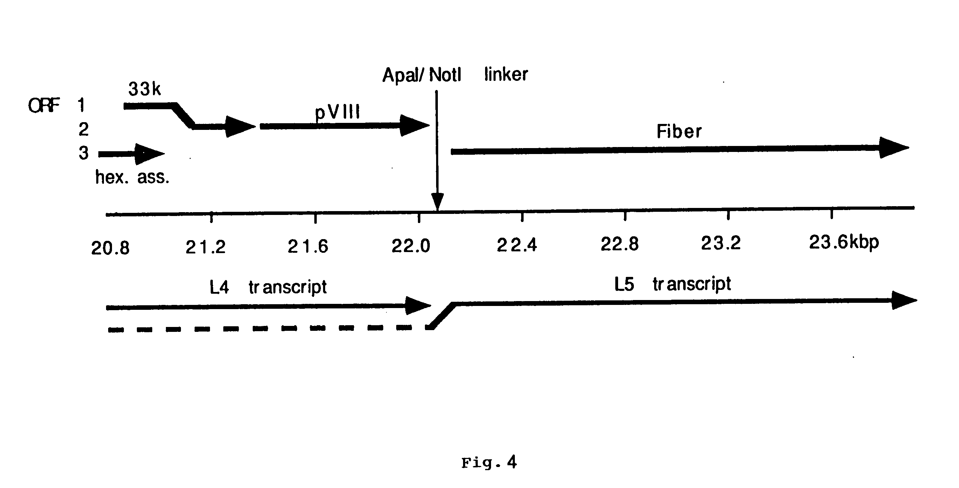

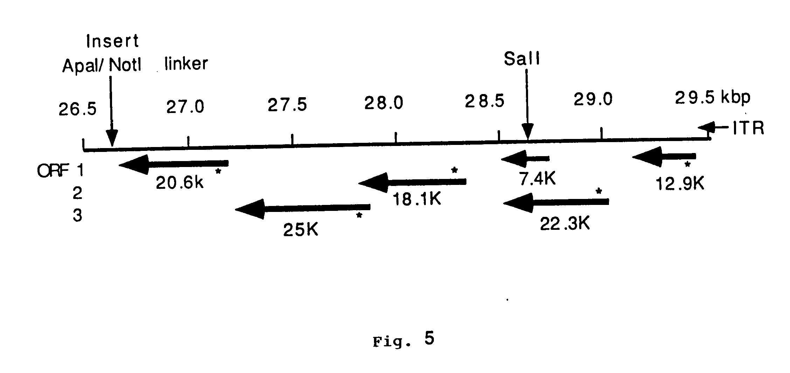

The viral sequence of 29,544 nucleotides (Figure 1) is considerably shorter (by approximately 6.5kb) than the sequence for human adenoviruses but many genes encoding structural proteins are identified by their homology with their Ad2 homologues (Figure 2). It is clear, however, that the ovine adenovirus genome shows major structural and sequence variations compared with all other adenoviruses studied to date (Figure 2), in the regions encoding both structural and non-structural proteins. In particular,
(a) the reading frames tentatively identified as forming the E1A/B regions are named principally on the basis of their location in the genome. Very limited homology can be detected between the 44.5kD open reading frame (orf) and the large T E1B protein of other adenoviruses. Homology in the putative E1A region of OAV287 has not so far been detected;
(b) in other adenoviruses the E4 region is normally located at the right-hand end of the genome. The OAV287 E4? region is tentatively identified based only on the presence of a protein sequence motif HCHC..PGSLQC which is found in 18.8kD and 30.85kD orfs in this region. Identical or very similar motifs are found in the E4 34kD protein of human Ad2 and Ad40 and mouse adenoviruses;
(c) the distance between the end of pVIII and the beginning of fiber, which in other viruses defines the E3 region, is only 197 nucleotides (Figure 4). The E3 region equivalent, if it exists in ovine adenovirus, may consist of the cluster of open reading frames which are present in the right to left orientation on the complementary DNA strand, at the right-hand end of the genome (Figures 2 and 5). However, these sequences show no detectable homology with any other adenovirus and the functions of these proteins cannot be deduced from such comparisons;
(d) there is a region of approximately 1kb which lies between E3? and E4? which has a very high A/T content (70.2%) (Figure 1). As there are no open reading frames encoding greater than approximately 30 amino acids in length on either DNA strand it is unlikely that the region codes for any proteins, unless mRNAs are generated by very complex splicing events. This region has no known equivalent in any other adenovirus;
(e) other differences are apparent in the structural proteins of the virus. OAV287 lacks homologues of Ad2 proteins V and IX. However, OAV287 has a completely new gene coding for p28kD which is located on the complementary strand of the E1A? region (Figure 2 and 3). This is a structural protein with an apparent size of 28kD by SDS PAGE which, according to N-terminal sequencing data, is cleaved from a larger precursor. No homology between this protein and others in the databases has been detected;
(f) in most other genomes the VA RNA genes are located between the Terminal protein and the 52/55k genes. In OAV287 there is no room for them as the reading frames overlap.

These differences serve to emphasize the unique character of the OAV287 isolate compared with other human and animal adenoviruses. In addition, since the OAV287 non-structural regions show little or no homology with equivalent regions in other adenoviruses, sequence comparisons do not reveal the identity of likely non-essential regions of the genome. Moreover the viral DNA cannot easily be manipulated to test for dispensable sequences.

The present inventors have produced a plasmid containing a full length infectious copy of an ovine adenovirus genome in which the ITR sequences are linked by a short sequence which creates a unique restriction enzyme site. A plasmid containing a full length infectious copy of an ovine adenovirus genome linked to a bacterial origin for DNA replication and a marker gene has been produced. Partial clones of OAV287 genomic DNA were specifically modified and initially linked to a gene encoding antibiotic resistance and origin of replication inserted into the unique SalI site of the genome (Figure 6 and see Methods). Such a plasmid can be grown in bacteria and more easily manipulated.

The circular genomic clone differs from the naturally occurring circles that occur in Ad5-infected cells (10) and that might exist in OAV287-infected cells in that the 40 base pair ITRs are joined by a GTAC linker. Together with the last and first nucleotides of the genome (G and C, respectively, see Figure 1), this sequence forms a unique KpnI site (GGTACC) when the ITRs are joined head to tail. Other sites such as EcoRI, BamHI, SalI, KasI etc which have recognition sequences beginning with G and ending with C are suitable if they are unique as the 3' and 5' terminal nucleotides of other adenovirus genomes are G and C, respectively. A plasmid with a suitable antibiotic resistance gene e.g. ampR and origin of replication can be inserted at the unique site or elsewhere in the genome to form a plasmid which can be propagated in bacteria. Plasmids propagated in the presence of 200 µg/ml ampicillin in E.coli strains JM109 and DH5-alpha retain the KpnI sites and inserted sequences, indicating that the OAV287 ITR sequences are stable when linked in this manner. This approach may therefore be used to engineer other adenovirus genomes. If desired the GTAC linker sequence can be removed and the authentic termini regenerated prior to transfection by digestion with KpnI (or another appropriate enzyme) and incubation with T4 DNA polymerase to create blunt ends (9).

A method for generating linear infectious genomes from circular plasmids involved digesting the circular plasmid containing the full length copy of the OAV287 genome with restriction enzyme KpnI to generate a genome with the authentic 5' nucleotide dCMP. The linear DNA is then introduced into CSL503 cells using lipofectamine as the transfecting reagent.

To develop a viral genome as a vector it is essential to identify region(s) of the genome which are non-essential for function. These regions can be then substituted or deleted to make room for foreign DNA (11, 12), or they may be the site for insertion of foreign DNA. In the human adenovirus genome DNA has been substituted or inserted into the E1 and E3 regions (13, 14, 15) and at the extreme right-hand end of the genome between E4 and ITR, usually with the concomitant deletion of non-essential regions to facilitate packaging of the genome (16). Adenoviruses will package genomes up to ~6% larger than the wild-type, probably due to physical constraints dictated by the capsid structure (11).

Non-essential sites in the OAV287 genome were identified by insertion of a polylinker sequence containing ApaI and NotI restriction sites. This linker was introduced into the genome copy in pOAV100 between nucleotides 22,139 and 22,130 of Figure 1 by site directed mutagenesis to create plasmid pOAV200 (Figure 7). This corresponds to a site located in the intergenic region between genes for the pVIII and fiber proteins which was chosen because it avoids disruption of RNA processing signals in the region. A transcription termination site for the L4 family of RNAs maps 26 nucleotides upstream and the splice junction between
the tripartite leader sequences and fiber mRNA maps 144 nucleotides downstream of the insertion site, respectively (17). Transfection of pOAV200 into CSL503 cells resulted in the rescue of virus OAV200. The second site at which the polylinker was inserted was located between bases 26,645 and 26,646 of Figure 1. This created plasmid pOAV600 (Figure 7). This insertion site corresponds to the right hand end of the A/T-rich region (Figure 2) whose function and precise boundaries are unknown. The site was chosen as it is six nucleotides to the left of the transcription termination point for RNAs transcribed from right to left from the E3? region (Figure 2). This was determined by sequencing cloned RT-PCR-amplified cDNAs derived from the region using methods similar to those described for the pVIII/fiber region (17). Transfection of pOAV600 into CSL503 cells yielded virus OAV600.

The above insertion strategy identified two regions of the genome which can be interrupted and created sites for subcloning gene expression cassettes.

A further non-essential site was identified using the unique SalI site located at bases 28644-28649 of Figure 1. The site was cut with SalI, end-filled and religated to disrupt the reading frames which spanned the site. A plasmid pOAV600S (Figure 7), which had lost the site was identified by digestion with SalI. When pOAV600S was transfected into CSL503 cells, virus OAV600S was recovered. The loss of the SalI site in this virus was confirmed by digesting the viral genome with SalI. As the SalI site falls within two significant open reading frames (which extend on the complementary strand between bases 28457 and 29014 and between 28511 and 28699), which were disrupted by end-filling and religation, the gene products derived from the reading frames are probably also dispensable. This group of reading frames may therefore constitute the E3 region of OAV287 as no other gene products in any adenovirus are dispensable for replication, in vitro. This implies that it should be possible to delete the whole region labelled as E3? in Figure 2. In addition, in other experiments a 1kb NdeI fragment was deleted from the region marked as E4? in Figure 2. This deletion disrupted several reading frames in the region. No virus has been rescued from a such a plasmid, suggesting that it is not dispensable and accordingly, it may be E4.

Many viruses replicate incompletely in heterologous hosts, often entering cells but being unable to produce mature virus particles because of a block in the replication cycle. In the context of recombinant viral vectors, this represents a desirable safety feature, provided that replication is not blocked before appropriate and effective expression of the foreign gene occurs. OAV287 does not replicate productively in heterologous cell types (18), the only exception so far being bovine nasal turbinate cells in which viral titres are significantly reduced compared with the CSL503 cells. Recombinant forms of OAV287 have been constructed to determine whether expression of a reporter gene under the control of an appropriate promoter occurs.

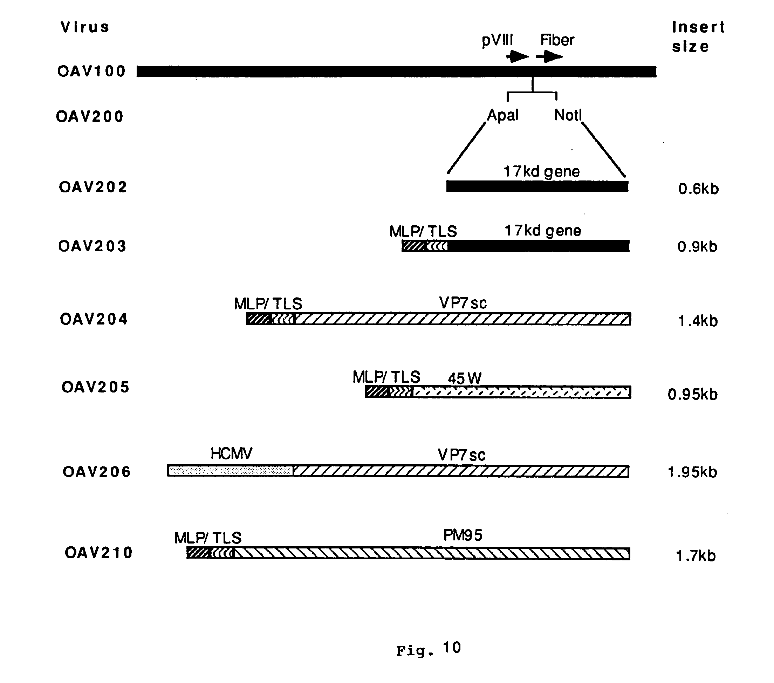

Foreign gene expression requires that the gene be functionally linked to a promoter. This may be a viral promoter inherent in the genome, or a foreign promoter subcloned together with the gene of interest into a suitable site. The promoter driving gene expression must function in CSL503 and preferably a range of other cell types. In this work an OAV287 genomic promoter was used initially. Subsequently an heterologous promoter was also used. In adenoviruses, expression of the structural proteins is driven by the major late promoter (MLP). Families of RNA transcripts derived from the MLP contain a common sequence element, the tripartite leader sequence (TLS) at their 5' ends. The present inventors have identified those nucleotides in the OAV287 genome which comprise the TLS by using RT-PCR amplification of late mRNA transcripts present in OAV287-infected cells and sequencing of cloned cDNAs (17). A candidate MLP was expected to be present just to the left of TLS exon 1 (Figure 2). The MLP and TLS elements were subcloned using PCR techniques into a separate plasmid pMT (Figure 9) and linked with genes of interest. These promoter/gene cassettes were subcloned as ApaI/NotI fragments into the polylinker ApaI/NotI sites of pOAV200. Using this strategy plasmids pOAV203, pOAV204, pOAV205 and pOAV210 were constructed. These incorporate genes encoding a 17kD soluble protein from *T. colubriformis,* a rotavirus VP7sc gene (19), the 45W antigen from *Taenia ovis* (20) and a membrane protein (PM95) from *Lucilia cuprina,* respectively. Plasmid pOAV202, contained the 17kD antigen but lacked the MLP/TLS elements. These plasmids were transfected into CSL503 cells and rescued as viruses OAV202, OAV203, OAV204, OAV205 and OAV210, respectively (Figure 10).

The human cytomegalovirus immediate early IE94 promoter plus enhancer, which functions in a range of human and animal cell types (21), was also linked to the rotavirus VP7sc antigen gene. This cassette was assembled by replacing the MLP/TLS elements in pMT/VP7sc with the HCMV enhancer-promoter region. The cassette was inserted in pOAV200 to create pOAV206. pOAV206 was transfected into CSL503 cells and virus OAV206 was rescued (Figure 10).

CSL503 and other cells were infected with the viruses described above and at various times post-infection the cells were radiolabelled with ³⁵S-methionine. Proteins of interest were recovered from cell lysates by immunoprecipitation using an appropriate antiserum. Recovered proteins were analysed by polyacrylamide gel electrophoresis and detected by autoradiography.

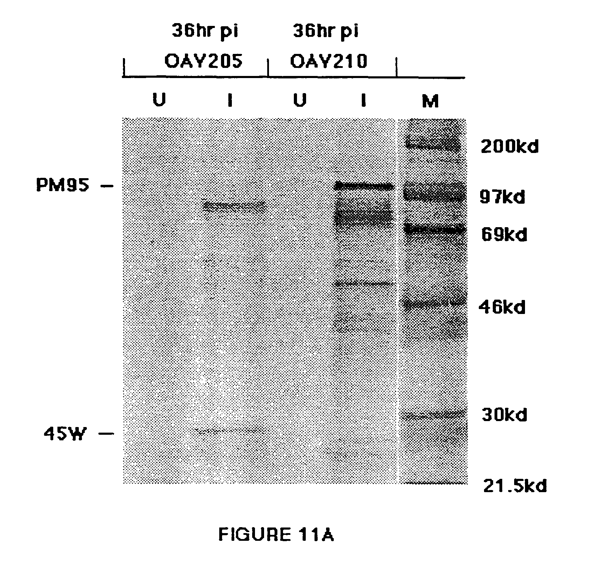

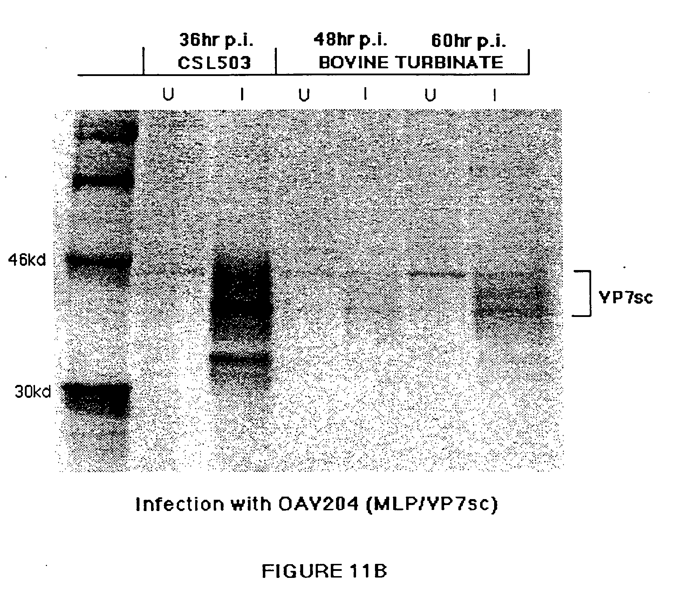

When virus OAV202 was used, no expression of the T. *coulbriformis* 17kD antigen was observed by immunofluorescence. As this virus lacks the MLP/TLS elements and carries only the 17kD gene this result demonstrates that there is no viral promoter upstream or adjacent to the insertion point between the pVIII and fiber genes which is capable of driving gene expression. As the E3 region is also missing from this site there is no requirement for a nearby promoter. This situation contrasts with observations made using a human Ad5 E3 recombinant (21). In this case a promoterless gene inserted 3' proximal to the pVIII gene was expressed, probably from the adjacent E3 promoter or the upstream MLP (15, 21). This result further emphasizes the unique nature of the OAV287 genome. Recombinant OAV287 viruses carrying the MLP/TLS elements were tested for expression in CSL503 cells. With OAV204, expression was easily detected in infected, but not in uninfected cells at 24hr post-infection (Figure 11A). Similarly, when viruses OAV205, and OAV210 were tested, gene products of 24kD and approximately 95kD, respectively were detected (Figure 11B). Therefore it is clear that MLP/TLS elements contain the necessary information to drive gene expression in the homologous cell line under replication-permissive conditions. However, when OAV204 was tested in a heterologous rabbit kidney cell line in which the virus does not replicate productively, no VP7sc expression was observed. Some replication occurs in bovine nasal turbinate cells, although to a lower titre than in CSL503 cells. In the latter cells, expression of VP7sc was detected following infection with OAV204 (Figure 11B).

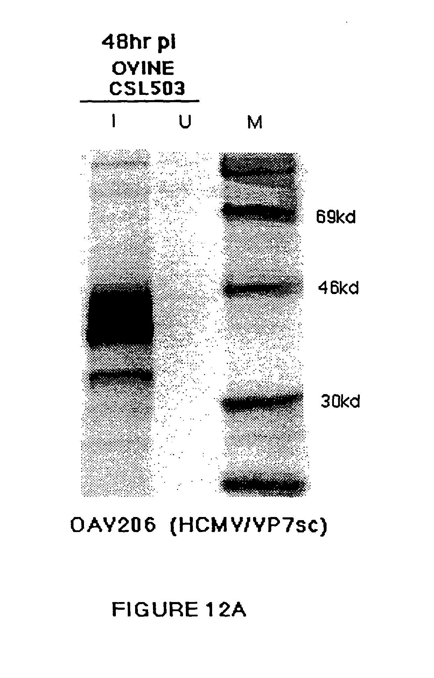

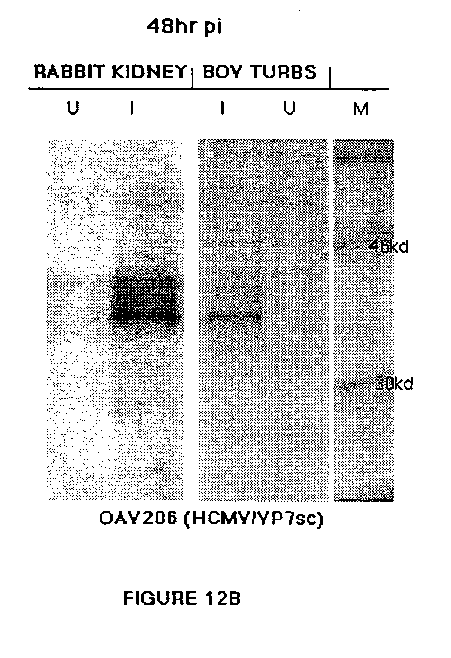

Virus OAV206 containing the HCMV enhancer/promoter element linked to the VP7sc gene was used to examine the function of a heterologous promoter in the context of the OAV287 genome. CSL503 cells infected with this virus readily expressed VP7sc antigen at 24-48hr post infection (Figure 12A). With this virus VP7sc expression was also observed in the non-permissive rabbit kidney cell line and in bovine nasal turbinate cells (Figure 12B). These results suggest that the HCMV or a similar constitutive promoter may be preferred over the MLP to drive gene expression in OAV recombinants in non-permissive cells.

One recombinant virus was also administered to sheep. Five sheep were vaccinated intraconjunctivally and intranasally with 0.7x10⁸ pfu of OAV203. At three days post-inoculation virus was recovered from the nasal swab of one sheep and from the conjunctival swabs of two sheep and confirmed as the recombinant virus by PCR analysis. Animals showed no obvious ill effects from such vaccination.

The viral vectors of the present invention can be used for the delivery and expression of therapeutic genes in grazing animals. In species which are not normally infected by ovine adenoviruses the lack of pre-existing immunity should allow efficient infection, gene delivery and expression. The genes may encode vaccine antigens, molecules which promote growth in production animals, molecules which modify production traits by manipulating hormone responses and other biologically active or therapeutic molecules. The virus does not replicate productively in many non-ovine cells but the use of heterologous promoters allows the delivery and expression of genes while minimising the possibility of virus spread to a non-target host. As the DNA of adenovirus vectors can persist in cells in an unintegrated form, with the appropriate choice of promoter, expression over a prolonged period can be achieved.

### References

1. R. B. Couch, et al., Am Rev Resp Dis 88, 394-403 (1963).
2. T. Ragot, et al., Nature 361, 647-650 (1993).
3. M. S. Horwitz, in Virology B. N. Fields, D. M. Knipe, Eds. (Raven Press, New York, 1990) pp. 1679-1721.
4. W. S. M. Wold, L. R. Gooding, Virology 184, 1-8 (1991).
5. K. S. Raviprakash, A. Grunhaus, M. A. El Kholy, M. S. Horwitz, J Virol 63, 5455-5458 (1989).
6. A. M. Lewis, et al., J Virol 11, 655-664 (1973).
7. R. L. Peet, W. Coackley, D. A. Purcell, C. W. Robartson, B. M. Micke, Aust Vet J 60, 307-308 (1983).
8. M. Benko, A. Bartha, G. Wadell, Intervirol 29, 346-350 (1988).
9. J. Sambrook, E. F. Fritsch, T. Maniatis, Eds., Molecular Cloning: A Laboratory Manual (Cold Spring Harbor Laboratory Press, Cold Spring Harbor, 1989).
10. F. L. Graham, EMBO J 3, 2917-2922 (1984).
11. F. L. Graham, L. Prevec, in Methods in Molecular Biology E. J. Murray, J. M. Walker, Eds. (Humana Press, Clifton, NJ, 1991), vol. 7, pp. 1-19.
12. F. L. Graham, L. Prevec, in Vaccines: New approaches to immunological problems R. W. Ellis, Ed. (Butterworth-Heinemann, Stoneham, Ma, 1992) pp. 363-390.
13. M. Levrero, et al., GENE (Amst) 101, 195-202 (1991).
14. J. E. Morin, et al., Proc. Natl. Acad. Sci. USA 84, 4626-4630 (1987).
15. G. W. Both, et al., Virology 193, 940-950 (1993).
16. P. K. Chanda, et al., Virology 175, 535-547 (1990).
17. S. V. Vrati, D. B. Boyle, R. Koekerhans, G. W. Both, Virology 209, In press, (1995).
18. D. B. Boyle, et al., Vet Microbiol 41, 281-291 (1994).
19. M. E. Andrew, et al., J Virol 64, 4776-4783 (1990).
20. K. S. Johnson, et al., Nature 338, 505-587 (1989).
21. Z. Z. Xu, V. Krougliak, L. Prevec, F. L. Graham, G. W. Both, J gen Virol In Press, (1995).
22. S. H. Larsen, H. D, Virology 82, 182-195 (1977).
23. E. Nakano, D. Panicali, E. Paoletti, Proc Natl Acad Sci USA 79, 1593-1596 (1982).
24. D. M. K. Rekosh, W. C. Russell, A. J. D. Bellet, A. J. Robinson, Cell 11, 2B3-295 (1977).
25. F. Sanger, S. Nicklen, A. R. Coulson, Proc Natl Acad Sci USA 74, 5463-5467 (1977).

## Claims

1. A recombinant ovine adenovirus comprising at least one nucleic acid sequence encoding a non-adenoviral polypeptide, wherein the recombinant adenovirus comprises the nucleic acid sequence as shown in Figure 1.

2. A recombinant ovine adenovirus according to claim 1, wherein the nucleic acid sequence encoding the non-adenoviral polypeptide encodes a polypeptide derived from bacteria, viruses, parasites or eukaryotes.

3. A recombinant ovine adenovirus according to claim 2, wherein the non-adenoviral polypeptide is rotavirus VP7sc antigen or *Trichostrongylus colubriformis* 17 kD antigen.

4. A method of delivering a nucleic acid encoding a non-adenoviral polypeptide to a target cell *in vitro,* the method comprising infecting the target cell with a recombinant ovine adenovirus according to any one of claims 1 to 3 such that the nucleic acid sequence encoding the non-adenoviral polypeptide is expressed by the target cell.

5. A recombinant ovine adenovirus according to any one of claims 1 to 3 for use in delivery of the non-adenoviral polypeptide to an animal.

6. The recombinant ovine adenovirus according to claim 5, wherein the animal is a grazing animal.

7. The recombinant ovine adenovirus according to claim 6, wherein the grazing animal is a sheep.

8. A recombinant ovine adenovirus according to claim 1 comprising at least one nucleic acid sequence encoding a functional RNA molecule.

9. A recombinant ovine adenovirus according to claim 8, wherein the functional RNA molecule is an antisense RNA molecule or a ribozyme.

10. A method of delivering a functional RNA molecule to a target cell *in vitro,* the method comprising infecting the target cell with a recombinant ovine adenovirus according to claim 8 or claim 9 such that the functional RNA molecule is expressed by the target cell.

11. A recombinant ovine adenovirus according to claim 8 or claim 9 for use in delivery of the functional RNA molecule to a target cell.

12. An isolated DNA molecule derived from an ovine adenovirus as claimed in claim 1, wherein a non-essential region is deleted or altered.

13. An isolated DNA molecule derived from an ovine adenovirus as claimed in claim 1, wherein the DNA molecule comprises at least one nucleic acid sequence encoding a non-adenoviral polypeptide, or at least one functional RNA molecule, inserted in a region of the DNA molecule corresponding to the SalI site between nucleotides 28,643 and 28,648 as shown in Figure 1.

14. An isolated DNA molecule comprising an inverted terminal repeat having the nucleic acid base sequence of 1 to 46 as shown in Figure 1.

15. A plasmid comprising the DNA molecule according to any one of claims 12 to 14.

16. A plasmid according to claim 15 which further comprises a nucleic acid sequence encoding an origin of replication and a further nucleic acid sequence encoding a marker.

17. A plasmid according to claim 16, wherein the marker encodes for resistance to an antimicrobial agent.

18. A recombinant ovine adenovirus according to any one of claims 1 to 3 for use in delivering a nucleic acid encoding a non-adenoviral polypeptide to a target cell.

## Patentansprüche

1. Rekombinantes ovines Adenovirus, das wenigstens eine Nucleinsäuresequenz aufweist, die für ein nicht-adenovirales Polypeptid codiert, wobei das rekombinante Adenovirus die Nucleinsäuresequenz umfasst, wie sie in Figur 1 gezeigt ist.

2. Rekombinantes ovines Adenovirus nach Anspruch 1, wobei die Nucleinsäuresequenz, die für das nicht-adenovirale Polypeptid codiert, für ein Polypeptid codiert, das sich ableitet von Bakterien, Viren, Parasiten oder Eukaryonten.

3. Rekombinantes ovines Adenovirus nach Anspruch 2, wobei das nicht-adenovirale Polypeptid das Rotavirus VP7sc-Antigen oder *Trichostrongylus colubriformis* 17kD-Antigen ist.

4. Verfahren zur Übertragung einer Nucleinsäure, die für ein nicht-adenovirales Polypeptid codiert, auf eine Zielzelle *in vitro,* wobei das Verfahren das Infizieren der Zielzelle mit einem rekombinanten ovinen Adenovirus nach irgendeinem der Ansprüche 1 bis 3 umfasst, so dass die Nucleinsäuresequenz, die für das nicht-adenovirale Polypeptid codiert, von der Zielzelle exprimiert wird.

5. Rekombinantes ovines Adenovirus nach irgendeinem der Ansprüche 1 bis 3 zur Verwendung bei der Einführung des nicht-adenoviralen Polypeptids in ein Tier.

6. Rekombinantes ovines Adenovirus nach Anspruch 5, wobei das Tier ein grasendes Tier ist.

7. Rekombinantes ovines Adenovirus nach Anspruch 6, wobei das grasende Tier ein Schaf ist.

8. Rekombinantes ovines Adenovirus nach Anspruch 1, das wenigstens eine Nucleinsäuresequenz umfasst, die für ein funktionales RNA-Molekül codiert.

9. Rekombinantes ovines Adenovirus nach Anspruch 8, wobei das funktionale RNA-Molekül ein Antisense-RNA-Molekül oder ein Ribozym ist.

10. Verfahren zur Einführung eines funktionalen RNA-Moleküls in eine Zielzelle *in vitro*, wobei das Verfahren das Infizieren der Zielzelle mit einem rekombinanten ovinen Adenovirus nach Anspruch 8 oder Anspruch 9 umfasst, so dass das funktionale RNA-Molekül von der Zielzelle exprimiert wird.

11. Rekombinantes ovines Adenovirus nach Anspruch 8 oder Anspruch 9 zur Verwendung bei der Einführung des funktionalen RNA-Moleküls in eine Zielzelle.

12. Isoliertes DNA-Molekül, das sich von einem ovinen Adenovirus ableitet, wie es in Anspruch 1 beansprucht wird, worin ein nicht-essentieller Bereich eliminiert oder verändert ist.

13. Isoliertes DNA-Molekül, das sich von einem ovinen Adenovirus ableitet, wie es in Anspruch 1 beansprucht wird, wobei das DNA-Molekül wenigstens eine Nucleinsäuresequenz umfasst, die für ein nicht-adenovirales Polypeptid codiert, oder für wenigstens ein funktionales RNA-Molekül, die in einen Bereich des DNA-Moleküls inseriert sind, der der SalI-Stelle zwischen den Nucleotiden 28.643 und 28.648, wie sie in Fig. 1 gezeigt sind, entspricht.

14. Isoliertes DNA-Molekül, das einen invertierten terminalen Repeat umfasst, der die Nucleinsäure-Basensequenz von 1 bis 46 aufweist, wie sie in Figur 1 gezeigt ist.

15. Plasmid, das das DNA-Molekül nach irgendeinem der Ansprüche 12 bis 14 umfasst.

16. Plasmid nach Anspruch 15, dass außerdem eine Nucleinsäuresequenz umfasst, die für einen Replikationsursprung codiert, sowie eine weitere Nucleinsäuresequenz, die für einen Marker codiert.

17. Plasmid nach Anspruch 16, wobei der Marker für eine Widerstandsfähigkeit gegen ein antimikrobielles Mittel codiert.

18. Rekombinantes ovines Adenovirus nach irgendeinem der Ansprüche 1 bis 3 zur Verwendung bei der Einführung einer Nucleinsäure, die für ein nicht-adenovirales Polypeptid codiert, in eine Zielzelle.

## Revendications

1. Adénovirus ovin recombinant comprenant au moins une séquence d'acide nucléique codant pour un polypeptide non adénoviral, où l'adénovirus recombinant comprend la séquence d'acide nucléique telle que représentée sur la figure 1.

2. Adénovirus ovin recombinant selon la revendication 1. dans lequel la séquence d'acide nucléique codant pour le polypeptide non adénoviral code pour un polypeptide dérivé de bactéries, de virus, de parasites ou d'eucaryotes.

3. Adénovirus ovin recombinant selon la revendication 2, dans lequel le polypeptide non adénoviral est l'antigène VP7sc de rotavirus ou l'antigène de 17 kD de *Trichostrongylus colubriformis.*

4. Procédé de délivrance d'un acide nucléique codant pour un polypeptide non adénoviral à une cellule cible *in vitro,* le procédé comprenant l'infection de la cellule cible avec un adénovirus ovin recombinant selon l'une quelconque des revendications 1 à 3 de telle manière que la séquence d'acide nucléique codant pour le polypeptide non adénoviral soit exprimée par la cellule cible.

5. Adénovirus ovin recombinant selon l'une quelconque des revendications 1 à 3, pour une utilisation dans la délivrance du polypeptide non adénoviral à un animal.

6. Adénovirus ovin recombinant selon la revendication 5. où l'animal est un animal de pâturage.

7. Adénovirus ovin recombinant selon la revendication 6. où l'animal de pâturage est un mouton.

8. Adénovirus ovin recombinant selon la revendication 1 comprenant au moins une séquence d'acide nucléique codant pour une molécule d'ARN fonctionnel.

9. Adénovirus ovin recombinant selon la revendication 8. où la molécule d'ARN fonctionnel est une molécule d'ARN antisens ou un ribozyme.

10. Procédé de délivrance d'une molécule d'ARN fonctionnel à une cellule cible *in vitro,* le procédé comprenant l'infection de la cellule cible avec un adénovirus ovin recombinant selon la revendication 8 ou la revendication 9 de telle manière que la molécule d'ARN fonctionnel soit exprimée par la cellule cible.

11. Adénovirus ovin recombinant selon la revendication 8 ou la revendication 9 pour une utilisation dans la délivrance de la molécule d'ARN fonctionnel à une cellule cible.

12. Molécule d'ADN isolé dérivée d'un adénovirus ovin selon la revendication 1, dans laquelle une région non essentielle est délétée ou modifiée.

13. Molécule d'ADN isolé dérivée d'un adénovirus ovin selon la revendication 1, où la molécule d'ADN comprend au moins une séquence d'acide nucléique codant pour un polypeptide non adénoviral, ou au moins une molécule d'ARN fonctionnel, insérée dans une région de la molécule d'ADN correspondant au site SalI entre les nucléotides 28643 et 28648 tel que représente sur la figure 1.

14. Molécule d'ADN isolé comprenant une répétition terminale inversée ayant la séquence des bases d'acide nucléique de 1 à 46 telle que représentée sur la figure 1.

15. Plasmide comprenant la molécule d'ADN selon l'une quelconque des revendications 12 à 14.

16. Plasmide selon la revendication 15 qui comprend en outre une séquence d'acide nucléique codant pour une origine de réplication et une autre séquence d'acide nucléique codant pour un marqueur.

17. Plasmide selon la revendication 16, où le marqueur code pour une résistance à un agent antimicrobien.

18. Adéliovii-tiq ovin recombinant selon l'une quelconque des revendications 1 à 3 pour une utilisation dans la délivrance d'un acide nucléique codant pour un polypeptide non adénoviral à une cellule cible.
